# EUROPEAN PATENT SPECIFICATION

(11) **EP 3 976 764 B1**
(45) Date of publication and mention of the grant of the patent: **23.07.2025**
(21) Application number: 20815471.6
(22) Date of filing: 29.05.2020
(51) Int. Cl.: C12N 5/0783, C07K 14/35, A61K 39/04, G01N 33/53

(54) **METHODS FOR DETECTING A MYCOBACTERIUM TUBERCULOSIS INFECTION**
VERFAHREN ZUM ERKENNEN EINER INFEKTION MIT DEM MYCOBAKTERIUM TUBERCULOSIS
MÉTHODES DE DÉTECTION D'UNE INFECTION PAR MYCOBACTERIUM TUBERCULOSIS

(30) Priority: 30.05.2019 US 201962854840 P
(43) Date of publication of application: 06.04.2022
(73) Proprietor: VITI, INC., Portland, OR 97201 (US); Oregon Health & Science University, Portland, OR 97239 (US)
(72) Inventor: LEWINSOHN, Deborah, Portland, Oregon 97201 (US); NYENDAK, Melissa, Portland, Oregon 97207 (US); SWARBRICK, Gwendolyn, Portland, Oregon 97207 (US); MOONEY, Jeffrey, Portland, Oregon 97206 (US)
(74) Representative: Boult Wade Tennant LLP
(86) International application number: PCT/US2020/035359
(87) International publication number: WO 2020/243594

(56) References cited:
- WO-A2-2005/090988
- US-A1- 2005 208 594
- US-A1- 2011 183 342
- US-A1- 2011 183 342
- US-A1- 2011 236 411
- US-B2- 7 572 597
- KHANNA DIVYA ET AL: "Ensemble Technique for Prediction of T-cellEpitopes", INTERDISCIPLINARY SCIENCES: COMPUTATIONAL LIFE SCIENCES, INTERNATIONAL ASSOCIATION OF SCIENTISTS IN THE INTERDISCIPLINARY AREAS, CA, vol. 11, no. 4, 7 November 2018 (2018-11-07), pages 611 - 627, XP036935946, ISSN: 1913-2751, [retrieved on 20181107], DOI: 10.1007/S12539-018-0309-0

## Description

### INTRODUCTION

*Mycobacterium* is a genus of aerobic intracellular bacterial organisms that, upon infection of a host, survive within endosomal compartments of monocytes and macrophages. Human mycobacterial diseases include tuberculosis (caused by *M. tuberculosis*), leprosy (caused by *M. leprae*)*,* Bairnsdale ulcers (caused by *M. ulcerans*)*,* and various infections caused by *M. marinum, M. kansasii, M. scrofulaceum, M. szulgai, M. xenopi, M. fortuitum, M. chelonae, M. haemophilum* and *M. intracellulare* (see Wolinsky, Chapter 37 in Microbiology: Including Immunology and Molecular Genetics, 3rd Ed., Harper & Row, Philadelphia, 1980). Human *tuberculosis* caused by *Mycobacterium tuberculosis* (*M. tuberculosis*) is a severe global health problem, responsible for approx. 1.3 million deaths annually, according to the WHO.

Organisms of the *tuberculosis* complex can cause a variety of diseases, but the commonest route of invasion is by inhalation of bacteria. This initiates an infection in the lung, which can ultimately spread to other parts of the body. Normally, this infection is restricted in growth by the immune system and infected individuals have no symptoms. Approximately 30% of individuals are unable to contain the infection and they will develop primary disease, which in many cases will eventually prove fatal. However, it is believed that even those individuals who apparently control the infection remain infected, probably for the rest of their life. Certainly, individuals who have been healthy for years or even decades can suddenly develop tuberculosis, which has proven to be caused by the same organism they were infected with many years previously. *M. tuberculosis* and other organisms of the TB complex are unique in that the mycobacteria can evade the immune response and survive for long periods in a refractory nonreplicating or slowly-replicating stage. This is referred to as latent TB and is at present a very significant global health problem that is estimated to affect approximately ⅓ of the world's population (Anon., 2001). Kanna et al., 2018 relates to an ensemble technique for prediction of T-cell *Mycobacterium tuberculosis* epitopes (Khanna D, Rana PS. Ensemble Technique for Prediction of T-cell Mycobacterium tuberculosis Epitopes. Interdiscip Sci. 2019 Dec;11(4):611-627. doi: 10.1007/s12539-018-0309-0. Epub 2018 Nov 7. PMID: 30406342). US 2005/208594 Al relates to a method of diagnosing in an individual recent exposure to an agent which is a pathogen, vaccine or any other moiety which induces a cellular response, said method comprising determining in vitro whether the T cells of the individual recognise a protein from said agent having a length of at least 30 amino acids, to a greater extent than one or more peptide epitopes from the agent, a greater extent of recognition of the protein indicating that the individual has recently been exposed to the agent. US 2011/183342 Al relates to methods for detecting an infection with *Mycobacterium tuberculosis* (Mtb) in a subject, wherein the subject is a child, a subject with a latent *Mycobacterium tuberculosis* infection. WO 2005/090988 A2 relates to a method of diagnosing *Mycobacterium tuberculosis* infection in a human, or of determining whether a human has been exposed to *Mycobacterium tuberculosis.*

### SUMMARY

The invention provides a method for detecting an immune response to *Mycobacterium tuberculosis* in a subject, the method comprising:
contacting a biological sample comprising T cells from a subject suspected of having an immune response to *Mycobacterium tuberculosis* with at least one 8 to 18 amino acids long peptide comprising an amino acid sequence having at least 70% identity to the amino acid sequence:
   WMGPASMAMVAAAQP (SEQ ID NO: 3); and
detecting the presence or absence of T cells in the biological sample that specifically recognize the amino acid sequence of the at least one peptide.

In some embodiments, the at least one peptide is a single peptide comprising an amino acid sequence having at least 70% identity to the amino acid sequence set forth in SEQ ID NO: 3 or wherein the at least one peptide is a single peptide consisting of the amino acid sequence set forth in SEQ ID NO: 3.

In some embodiments, the method comprises contacting the biological sample with at least two peptides, at least three peptides, at least four peptides, at least five peptides, at least six peptides, at least seven peptides, at least eight peptides, at least nine peptides, at least ten peptides, at least eleven peptides, at least twelve peptides, at least thirteen peptides, or fourteen peptides, wherein at least one of said peptides is 8 to 18 amino acids long and comprises an amino acid sequence having at least 70% identity to the amino acid sequence: WMGPASMAMVAAAQP (SEQ ID NO: 3) or consists of the amino acid sequence set forth in SEQ ID NO: 3, and wherein the other peptide or peptides is/are 8 to 18 amino acids long and each independently comprise an amino acid sequence having at least 70% identity to the amino acid sequence set forth in SEQ ID NO:1, SEQ ID NO:2, SEQ ID NO:6, SEQ ID NO:7, SEQ ID NO:11, SEQ ID NO: 14, SEQ ID NO: 4, SEQ ID NO: 15, SEQ ID NO: 8, SEQ ID NO: 13, SEQ ID NO: 12, SEQ ID NO: 5, SEQ ID NO: 10 or QTVEDEARRMW (SEQ ID NO:9), or consist of the amino acid sequence set forth in SEQ ID NO:1, SEQ ID NO:2, SEQ ID NO:6, SEQ ID NO:7, SEQ ID NO:11, SEQ ID NO: 14, SEQ ID NO: 4, SEQ ID NO: 15, SEQ ID NO: 8, SEQ ID NO: 13, SEQ ID NO: 12, SEQ ID NO: 5 or SEQ ID NO: 10.

In some embodiments, the biological sample from the subject is blood, isolated peripheral blood mononuclear cells, or isolated mononuclear cells.

In some embodiments, the biological sample comprises antigen presenting cells.

In some embodiments, the T cells are CD8+ T cells.

In some embodiments, the detecting comprises measuring secretion of a cytokine from the CD8+ T cells and/or measuring an amount of a cytokine inside of the CD8+ T cells.

In some embodiments, the cytokine is interferon-γ, TNF, and/or IP-10.

In some embodiments, the detecting comprises contacting the CD8+ T cells with Tetrameric MHC Class I/peptide complexes that specifically associate with a CD8+ T cell surface protein.

In some embodiments, the detecting comprises conducting an enzyme-linked immunospot assay or an enzyme-linked immunosorbent assay, optionally wherein the assay detects interferon-γ or IP-10.

The invention also provides at least one 9 to 18 amino acids long peptide for use in a method of diagnosing an immune response to Mycobacterium tuberculosis in vivo, the method comprising:
administering into the skin of a subject suspected of having an immune response to Mycobacterium tuberculosis an effective amount of the at least one 9 to 18 amino acids long peptide, comprising an amino acid sequence having at least 70% identity to the amino acid sequence:
   WMGPASMAMVAAAQP (SEQ ID NO: 3); and
detecting presence of T cells that specifically recognize the amino acid sequence of the peptide in the subject.

In some embodiments, the method comprises administering into the skin of the subject an effective amount of a single peptide, or at least two peptides, at least three peptides, at least four peptides, at least five peptides, at least six peptides, at least seven peptides, at least eight peptides, at least nine peptides, at least ten peptides, at least eleven peptides, at least twelve peptides, at least thirteen peptides, or fourteen peptides, wherein at least one of said peptides is 9 to 18 amino acids long and comprises an amino acid sequence having at least 70% identity to the amino acid sequence: WMGPASMAMVAAAQP (SEQ ID NO: 3) or consists of the amino acid sequence set forth in SEQ ID NO: 3, and wherein the other peptide or peptides is/are 9 to 18 amino acids long and each independently comprise an amino acid sequence having at least 70% identity to the amino acid sequence set forth in SEQ ID NO:1, SEQ ID NO:2, SEQ ID NO:6, SEQ ID NO:7, SEQ ID NO:11, SEQ ID NO: 14, SEQ ID NO: 4, SEQ ID NO: 15, SEQ ID NO: 8, SEQ ID NO: 13, SEQ ID NO: 12, SEQ ID NO: 5 or SEQ ID NO: 10, or consist of the amino acid sequence set forth in SEQ ID NO:1, SEQ ID NO:2, SEQ ID NO:6, SEQ ID NO:7, SEQ ID NO:11, SEQ ID NO: 14, SEQ ID NO: 4, SEQ ID NO: 15, SEQ ID NO: 8, SEQ ID NO: 13, SEQ ID NO: 12, SEQ ID NO: 5 or SEQ ID NO: 10, optionally wherein the T cells are CD8+ T cells.

In some embodiments, the detecting the presence or absence of T cells comprises detecting a delayed type hypersensitivity reaction, optionally wherein the delayed type hypersensitivity reaction is measured by measuring redness, swelling, or induration of the skin at or adjacent the site of administration.

The invention also provides an immunoassay kit for detecting the presence of T cells in a biological sample of a subject or at an intradermal site of administration in the subject indicative of an immune response to Mycobacterium tuberculosis in the subject, the kit comprising at least one 9 to 18 amino acids long peptide comprising an amino acid sequence having at least 70% identity to the amino acid sequence:
WMGPASMAMVAAAQP (SEQ ID NO: 3).

In some embodiments, the kit comprises at least a single peptide, at least two peptides, at least three peptides, at least four peptides, at least five peptides, at least six peptides, at least seven peptides, at least eight peptides, at least nine peptides, at least ten peptides, at least eleven peptides, at least twelve peptides, or at least thirteen peptides, at least fourteen peptides, wherein at least one of said peptides is 9 to 18 amino acids long and comprises an amino acid sequence having at least 70% identity to the amino acid sequence: WMGPASMAMVAAAQP (SEQ ID NO: 3) or consists of the amino acid sequence set forth in SEQ ID NO: 3, and wherein the other peptide or peptides is/are 9 to 18 amino acids long and each independently comprise an amino acid sequence having at least 70% identity to the amino acid sequence set forth in SEQ ID NO:1, SEQ ID NO:2, SEQ ID NO:6, SEQ ID NO:7, SEQ ID NO:11, SEQ ID NO: 14, SEQ ID NO: 4, SEQ ID NO: 15, SEQ ID NO: 8, SEQ ID NO: 13, SEQ ID NO: 12, SEQ ID NO: 5 or SEQ ID NO: 10or consist of the amino acid sequence set forth in SEQ ID NO:1, SEQ ID NO:2, SEQ ID NO:6, SEQ ID NO:7, SEQ ID NO:11, SEQ ID NO: 14, SEQ ID NO: 4, SEQ ID NO: 15, SEQ ID NO: 8, SEQ ID NO: 13, SEQ ID NO: 12, SEQ ID NO: 5 or SEQ ID NO: 10, optionally wherein the peptide is immobilized on a solid support.

### BRIEF DESCRIPTION OF THE DRAWINGS

**FIG. 1** depicts the sensitivity and specificity for detecting an immune response to *M. tuberculosis* using peptides having SEQ ID NOs: 10, 16-19 and derived from an Mtb antigen MSL.
**FIG. 2** depicts the sensitivity and specificity for detecting an immune response to *M. tuberculosis* using peptides having SEQ ID NOs: 13, 20-23 and derived from an Mtb antigen ctpF.
**FIG. 3** depicts the sensitivity and specificity for detecting an immune response to *M. tuberculosis* using peptides having SEQ ID NOs: 15, 24-26 and derived from an Mtb antigen PE3.
**FIG. 4** depicts the sensitivity and specificity for detecting an immune response to *M. tuberculosis* using peptides having SEQ ID NOs: 6, 14, 28-30 derived from an Mtb antigen PE3.
**FIG. 5** depicts the sensitivity and specificity for detecting an immune response to *M. tuberculosis* using peptides having SEQ ID NOs: 7, 31-32 and derived from an Mtb antigen irtA.
**FIG. 6** depicts the sensitivity and specificity for detecting an immune response to *M. tuberculosis* using peptides having SEQ ID NOs: 8, 33-36 and derived from an Mtb antigen PE12.
**FIG. 7** depicts the sensitivity and specificity for detecting an immune response to *M. tuberculosis* using peptides having SEQ ID NOs: 11, 37-39 and derived from an Mtb antigen PE_PGRS50.
**FIG. 8** depicts the sensitivity and specificity for detecting an immune response to *M. tuberculosis* using peptides having SEQ ID NOs: 12, 40-43 and derived from an Mtb antigen PPE51.
**FIG. 9** depicts the sensitivity and specificity for detecting an immune response to *M. tuberculosis* using peptides having SEQ ID NOs: 3, 44-47 and derived from an Mtb antigen PPE 15.
**FIG. 10** depicts the sensitivity and specificity for detecting an immune response to *M. tuberculosis* using peptides having SEQ ID NOs: 5, 48-51 and derived from an Mtb antigen PPE68.
**FIG. 11** depicts the sensitivity and specificity for detecting an immune response to *M. tuberculosis* using detecting an immune response to *M. tuberculosis* using peptides having SEQ ID NOs: 9, 52-54 and derived from an Mtb antigen EsxJ.
**FIG. 12** depicts the sensitivity and specificity for detecting an immune response to *M. tuberculosis* using peptides having SEQ ID NOs: 2, 55-57 and derived from an Mtb antigen ctpF.
**FIG. 13** depicts the sensitivity and specificity for detecting an immune response to *M. tuberculosis* using peptides having SEQ ID NOs: 4, 58-61 and derived from an Mtb antigen PPE15.
**FIG. 14** depicts the sensitivity and specificity for detecting an immune response to *M. tuberculosis* using peptides having SEQ ID NOs: 1 and 62-64 derived from the indicated Mtb antigen.

### DETAILED DESCRIPTION

Methods for detecting an immune response to *Mycobacterium tuberculosis* in a subject are provided. The methods include contacting a biological sample comprising T cells from the subject with at least one 8 to 18 amino acids long peptide comprising an amino acid sequence having at least 70% identity to SEQ ID NO: 3 and detecting the presence or absence of T cells in the biological sample that specifically recognize the amino acid sequence of the at least one peptide, wherein the presence of T cells that specifically recognize the amino acid sequence of the at least one peptide indicates the subject has an immune response to *Mycobacterium tuberculosis.* An immune response to *Mycobacterium tuberculosis* indicates that the bacteria is present in the subject. The methods of the present disclosure may include treating a subject identified as having an immune response to *M. tuberculosis.* Also provided are a skin test and immunoassay kits for practicing the methods of the invention. In some embodiments, the peptide is 9-18 amino acids long.

Before exemplary aspects of the present invention are described, it is to be understood that this invention is not limited to particular aspects described, as such may, of course, vary. It is also to be understood that the terminology used herein is for the purpose of describing particular aspects only, and is not intended to be limiting.

Where a range of values is provided, it is understood that each intervening value, to the tenth of the unit of the lower limit unless the context clearly dictates otherwise, between the upper and lower limits of that range is also specifically disclosed. Each smaller range between any stated value or intervening value in a stated range and any other stated or intervening value in that stated range is encompassed within the invention. The upper and lower limits of these smaller ranges may independently be included or excluded in the range, and each range where either, neither or both limits are included in the smaller ranges is also encompassed within the invention, subject to any specifically excluded limit in the stated range. Where the stated range includes one or both of the limits, ranges excluding either or both of those included limits are also included in the invention.

Unless defined otherwise, all technical and scientific terms used herein have the same meaning as commonly understood by one of ordinary skill in the art to which this invention belongs. Although any methods and materials similar or equivalent to those described herein can be used in the practice or testing of the present invention, some potential and exemplary methods and materials may now be described. It is understood that the present disclosure supersedes any disclosure of a cited publication to the extent there is a contradiction.

It must be noted that as used herein and in the appended claims, the singular forms "a", "an", and "the" include plural referents unless the context clearly dictates otherwise. Thus, for example, reference to "a T cell" includes a plurality of such T cells and reference to "the T cell" includes reference to one or more T cells, and so forth.

It is further noted that the claims may be drafted to exclude any element which may be optional. As such, this statement is intended to serve as antecedent basis for use of such exclusive terminology as "solely", "only" and the like in connection with the recitation of claim elements, or the use of a "negative" limitation.

The publications discussed herein are provided solely for their disclosure prior to the filing date of the present application. Nothing herein is to be construed as an admission that the present invention is not entitled to antedate such publication by virtue of prior invention. Further, the dates of publication provided may be different from the actual publication dates which may need to be independently confirmed. To the extent such publications may set out definitions of a term that conflicts with the explicit or implicit definition of the present disclosure, the definition of the present disclosure controls.

As will be apparent to those of skill in the art upon reading this disclosure, each of the individual aspects described and illustrated herein has discrete components and features which may be readily separated from or combined with the features of any of the other several aspects. Any recited method can be carried out in the order of events recited or in any other order which is logically possible.

### DEFINITIONS

Amplification: Use of a technique that increases the number of copies of a nucleic acid molecule (e.g., a DNA or RNA molecule) in a specimen. An example of amplification is the polymerase chain reaction, in which a biological sample collected from a subject is contacted with a pair of oligonucleotide primers, under conditions that allow for the hybridization of the primers to a nucleic acid template in the sample. The primers are extended under suitable conditions, dissociated from the template, and then re-annealed, extended, and dissociated to amplify the number of copies of the nucleic acid. The product of amplification can be characterized by electrophoresis, restriction endonuclease cleavage patterns, oligonucleotide hybridization or ligation, and/or nucleic acid sequencing using standard techniques. Other examples of amplification include strand displacement amplification, as disclosed in U.S. Pat. No. 5,744,311; transcription-free isothermal amplification, as disclosed in U.S. Pat. No. 6,033,881; repair chain reaction amplification, as disclosed in WO 90/01069; ligase chain reaction amplification, as disclosed in EP-A-320 308; gap filling ligase chain reaction amplification, as disclosed in U.S. Pat. No. 5,427,930; and NASBA^{™} RNA transcription-free amplification, as disclosed in U.S. Pat. No. 6,025,134.

Animal: Living multi-cellular vertebrate organisms, a category that includes, for example, mammals and birds. The term mammal includes both human and non-human mammals. Similarly, the term "subject" includes both human and veterinary subjects.

Antibody: Immunoglobulin molecules and immunologically active portions of immunoglobulin molecules, i.e., molecules that contain an antigen binding site that specifically binds (immunoreacts with) an antigen, such as an Mtb polypeptide.

A naturally occurring antibody (e.g., IgG, IgM, IgD) includes four polypeptide chains, two heavy (H) chains and two light (L) chains interconnected by disulfide bonds. However, it has been shown that the antigen-binding function of an antibody can be performed by fragments of a naturally occurring antibody. Thus, these antigen-binding fragments are also intended to be designated by the term "antibody." Specific, non-limiting examples of binding fragments encompassed within the term antibody include (i) a Fab fragment consisting of the V_{L}, V_{H}, C_{L} and C_{H1} domains; (ii) an F_{d} fragment consisting of the V_{H} and C_{H1} domains; (iii) an Fv fragment consisting of the V_{L} and V_{H} domains of a single arm of an antibody, (iv) a dAb fragment (Ward et al., Nature 341:544-546, 1989) which consists of a V_{H} domain; (v) an isolated complementarity determining region (CDR); and (vi) a F(ab')₂ fragment, a bivalent fragment comprising two Fab fragments linked by a disulfide bridge at the hinge region.

Immunoglobulins and certain variants thereof are known and many have been prepared in recombinant cell culture (e.g., see U.S. Pat. Nos. 4,745,055 and 4,444,487; WO 88/03565; EP 256,654; EP 120,694; EP 125,023; Falkner et al., Nature 298:286, 1982; Morrison, J. Immunol. 123:793, 1979; Morrison et al., Ann. Rev. Immunol. 2:239, 1984).

Antigen: A compound, composition, or substance that can stimulate the production of antibodies or a T cell response in an animal, including compositions that are injected or absorbed into an animal. An antigen reacts with the products of specific humoral or cellular immunity, including those induced by heterologous immunogens. The term "antigen" includes all related antigenic epitopes. "Epitope" or "antigenic determinant" refers to a site on an antigen to which B and/or T cells respond. In one aspect, T cells respond to the epitope, when the epitope is presented in conjunction with an MHC molecule. Epitopes can be formed both from contiguous amino acids or noncontiguous amino acids juxtaposed by tertiary folding of a protein. Epitopes formed from contiguous amino acids are typically retained on exposure to denaturing solvents whereas epitopes formed by tertiary folding are typically lost on treatment with denaturing solvents. An epitope typically includes at least 3, and more usually, at least 5, about 9, or about 8-10 amino acids in a unique spatial conformation. Methods of determining spatial conformation of epitopes include, for example, x-ray crystallography and 2-dimensional nuclear magnetic resonance.

An antigen can be a tissue-specific antigen, or a disease-specific antigen. These terms are not exclusive, as a tissue-specific antigen can also be a disease-specific antigen. A tissue-specific antigen is expressed in a limited number of tissues, such as a single tissue. A tissue-specific antigen may be expressed by more than one tissue, such as, but not limited to, an antigen that is expressed in more than one reproductive tissue, such as in both prostate and uterine tissue. A disease-specific antigen is expressed coincidentally with a disease process. Specific non-limiting examples of a disease-specific antigen are an antigen whose expression correlates with, or is predictive of, tuberculosis. A disease-specific antigen can be an antigen recognized by T cells or B cells.

Antigen presenting cell (APC): A cell that can present an antigen to T cell, such that the T cells are activated. Dendritic cells (DCs) are the principle APCs involved in primary immune responses. Their major function is to obtain antigen in tissues, migrate to lymphoid organs and present the antigen in order to activate T cells.

When an appropriate maturational cue is received, DCs are signaled to undergo rapid morphological and physiological changes that facilitate the initiation and development of immune responses. Among these are the up-regulation of molecules involved in antigen presentation; production of pro-inflammatory cytokines, including IL-12, key to the generation of Th1 responses; and secretion of chemokines that help to drive differentiation, expansion, and migration of surrounding naive Th cells. Collectively, these up-regulated molecules facilitate the ability of DCs to coordinate the activation and effector function of other surrounding lymphocytes that ultimately provide protection for the host.

cDNA (complementary DNA): A piece of DNA lacking internal, non-coding segments (introns) and regulatory sequences that determine transcription. cDNA is synthesized in the laboratory by reverse transcription from messenger RNA extracted from cells.

CD4: Cluster of differentiation factor 4, a T cell surface protein that mediates interaction with the MHC Class II molecule. CD4 also serves as the primary receptor site for HIV on T cells during HIV infection. Cells that express CD4 are often helper T cells.

CD8: Cluster of differentiation factor 8, a T cell surface protein that mediates interaction with the MHC Class I molecule. Cells that express CD8 are often cytotoxic T cells. "CD8⁺ T cell mediated immunity" is an immune response implemented by presentation of antigens to CD8⁺ T cells.

Conservative variants: A substitution of an amino acid residue for another amino acid residue having similar biochemical properties. "Conservative" amino acid substitutions are those substitutions that do not substantially decrease an activity or antigenicity of the *Mycobacterium* peptide. A peptide can include one or more amino acid substitutions, for example 1-10 conservative substitutions, 2-5 conservative substitutions, 4-9 conservative substitutions, such as 1, 2, 5 or 10 conservative substitutions. Specific, non-limiting examples of a conservative substitution include the following examples (Table 1).

**TABLE 1**

| Exemplary conservative amino acid substitutions | |
|---|---|
| Original Amino Acid | Conservative Substitutions |
| Ala | Ser |
| Arg | Lys |
| Asn | Gln, His |
| Asp | Glu |
| Cys | Ser |
| Gln | Asn |
| Glu | Asp |
| His | Asn; Gln |
| Ile | Leu, Val |
| Leu | Ile; Val |
| Lys | Arg; Gln; Glu |
| Met | Leu; Ile |
| Phe | Met; Leu; Tyr |
| Ser | Thr |
| Thr | Ser |
| Trp | Tyr |
| Tyr | Trp; Phe |
| Val | Ile; Leu |

The term conservative variation also includes the use of a substituted amino acid in place of an unsubstituted parent amino acid, provided that antibodies raised to the substituted polypeptide also immunoreact with the unsubstituted polypeptide, or that an immune response can be generated against the substituted polypeptide that is similar to the immune response against the unsubstituted polypeptide, such as *a Mycobacterium* antigen. Thus, in one aspect, non-conservative substitutions are those that reduce an activity or antigenicity. Amino acid substitutions that do not significantly decrease antigenicity of a *M. tuberculosis* peptide may be determined based on the amino acid sequences of homologous proteins from different strains of *M. tuberculosis.* For example, amino acids that are not conserved in a protein present in different strains of *M. tuberculosis* may be substituted. The amino acid replacing a non-conserved amino acid in a *M*. *tuberculosis* peptide may be the amino acid present at the corresponding position in the protein from a different *M. tuberculosis* strain.

Consists Essentially Of/Consists Of: With regard to a polypeptide or peptide, a polypeptide or peptide consists essentially of a specified amino acid sequence if it does not include any additional amino acid residues. However, the polypeptide or peptide can include additional non-peptide components, such as labels (for example, fluorescent, radioactive, or solid particle labels), sugars or lipids. A polypeptide or peptide that consists of a specified amino acid sequence does not include any additional amino acid residues, nor does it include additional non-peptide components, such as lipids, sugars or labels.

Contacting: The process of incubating one agent in the presence of another. Thus, when a cell is contacted with an agent, the cell is incubated with the agent for a sufficient period of time for the agent and the cell to interact.

Degenerate variant: A polynucleotide encoding an epitope of an Mtb polypeptide that includes a sequence that is degenerate as a result of the genetic code. There are 20 natural amino acids, most of which are specified by more than one codon. Therefore, all degenerate nucleotide sequences are included in this disclosure as long as the amino acid sequence of the Mtb polypeptide encoded by the nucleotide sequence is unchanged.

Dendritic cell (DC): Dendritic cells are the principle APCs involved in primary immune responses. DCs include plasmacytoid dendritic cells and myeloid dendritic cells. Their major function is to obtain antigen in tissues, migrate to lymphoid organs and present the antigen in order to activate T cells. Immature DCs originate in the bone marrow and reside in the periphery as immature cells.

Diagnostic: Identifying the presence or nature of a pathologic condition, such as, but not limited to, tuberculosis. Diagnostic methods differ in their sensitivity and specificity. The "sensitivity" of a diagnostic assay is the percentage of diseased individuals who test positive (percent of true positives). The "specificity" of a diagnostic assay is 1 minus the false positive rate, where the false positive rate is defined as the proportion of those without the disease who test positive. While a particular diagnostic method may not provide a definitive diagnosis of a condition, it suffices if the method provides a positive indication that aids in diagnosis. "Prognostic" means predicting the probability of development (for example, severity) of a pathologic condition, such as tuberculosis.

Displaying: The process of localizing a peptide:: antigen complex, or a peptide, on the outer surface of a cell where the peptide::antigen complex or peptide is accessible to a second cell, molecules displayed by a second cell, or soluble factors. A peptide, or a peptide::antigen complex, is "displayed" by a cell when it is present on the outer surface of the cell and is accessible to a second cell, to molecules displayed by the second cell, or to soluble factors.

Epitope: An antigenic determinant. These are particular chemical groups or peptide sequences on a molecule that are antigenic, e.g., that elicit a specific immune response. An antibody specifically binds a particular antigenic epitope on a polypeptide, such as *a Mycobacterium* polypeptide.

Expression Control Sequences: Nucleic acid sequences that regulate the expression of a heterologous nucleic acid sequence to which it is operatively linked. Expression control sequences are operatively linked to a nucleic acid sequence when the expression control sequences control and regulate the transcription and, as appropriate, translation of the nucleic acid sequence. Thus, expression control sequences can include appropriate promoters, enhancers, transcription terminators, a start codon (e.g., ATG) in front of a protein-encoding gene, splicing signal for introns, maintenance of the correct reading frame of that gene to permit proper translation of mRNA, and stop codons. The term "control sequences" is intended to include, at a minimum, components whose presence can influence expression, and can also include additional components whose presence is advantageous, for example, leader sequences and fusion partner sequences. Expression control sequences can include a promoter.

A promoter is a minimal sequence sufficient to direct transcription. Also included are those promoter elements which are sufficient to render promoter-dependent gene expression controllable for cell-type specific, tissue-specific, or inducible by external signals or agents; such elements may be located in the 5' or 3' regions of the gene. Both constitutive and inducible promoters, are included (see e.g., Bitter et al., Meth. Enzymol. 153:516-544, 1987). For example, when cloning in bacterial systems, inducible promoters such as pL of bacteriophage lambda, plac, ptrp, ptac (ptrp-lac hybrid promoter) and the like may be used. In one aspect, when cloning in mammalian cell systems, promoters derived from the genome of mammalian cells (e.g., metallothionein promoter) or from mammalian viruses (e.g., the retrovirus long terminal repeat; the adenovirus late promoter; the vaccinia virus 7.5K promoter) can be used. Promoters produced by recombinant DNA or synthetic techniques may also be used to provide for transcription of the nucleic acid sequences. In one aspect, the promoter is a cytomegalovirus promoter.

Functionally Equivalent: Sequence alterations, such as in an epitope of an antigen, that yield the same results as described herein. Such sequence alterations can include, but are not limited to, conservative substitutions, deletions, mutations, frameshifts, and insertions.

Heterologous: Originating from separate genetic sources or species. A polypeptide that is heterologous to an Mtb polypeptide originates from a nucleic acid that does not encode the Mtb polypeptide or another Mtb polypeptide. In one specific, non-limiting example, a polypeptide comprising nine consecutive amino acids from an Mtb polypeptide, or at most 20 consecutive amino acids, from the Mtb polypeptide, and a heterologous amino acid sequence includes a β-galactosidase, a maltose binding protein, and albumin, hepatitis B surface antigen, or an immunoglobulin amino acid sequence. Generally, an antibody that specifically binds to a protein of interest will not specifically bind to a heterologous protein.

Host cells: Cells in which a vector can be propagated and its DNA expressed. The cell may be prokaryotic or eukaryotic. The cell can be mammalian, such as a human cell. The term also includes any progeny of the subject host cell. It is understood that all progeny may not be identical to the parental cell since there may be mutations that occur during replication. However, such progeny is included when the term "host cell" is used.

Human Leukocyte Antigen (HLA): A genetic designation of the human major histocompatibility complex (MHC). Individual loci are designated by uppercase letters, as in HLA-E, and alleles are designated by numbers, as in HLA-A*0201. The three main MHC class I genes are called HLA-A, HLA-B, and HLA-C. However, there are many genes that encode β2 microglobulin-associated cell surface molecules that are linked to the MHC class I genes. The expression of these genes is variable, both in the tissue distribution and the amount expressed on cells; these genes have been termed the MHC class IB genes.

Immune response: A response of a cell of the immune system, such as a B cell, natural killer cell, or a T cell, to a stimulus. In one aspect, the response is specific for a particular antigen (an "antigen-specific response"). In one aspect, an immune response is a T cell response, such as a Th1, Th2, or Th3 response. In another aspect, an immune response is a response of a suppressor T cell.

Immunogenic composition: A composition comprising an effective amount of an immunogenic Mtb polypeptide or a nucleic acid encoding the immunogenic Mtb polypeptide that induces a measurable T response against Mtb, such as a CD8⁺ T cell response, or induces a measurable B cell response (such as production of antibodies that specifically bind an Mtb polypeptide). For in vitro use, the immunogenic composition can consist of the isolated nucleic acid, vector including the nucleic acid/or immunogenic peptide. For in vivo use, the immunogenic composition will typically comprise the nucleic acid, vector including the nucleic acid, and/or immunogenic polypeptide in pharmaceutically acceptable carriers and/or other agents. An immunogenic composition can optionally include an adjuvant, a costimulatory molecule, or a nucleic acid encoding a costimulatory molecule. An Mtb polypeptide, or nucleic acid encoding the polypeptide, can be readily tested for its ability to induce a CD8⁺ T cell response.

Immunogenic peptide: A peptide which comprises an allele-specific motif or other sequence such that the peptide will bind an MHC molecule and induce a T cell response, such as a CD8⁺ or CD4⁺ T cell response, or a B cell response (such as antibody production) against the antigen from which the immunogenic peptide is derived.

In one aspect, immunogenic peptides are identified using sequence motifs or other methods, such as neural net or polynomial determinations, known in the art. Typically, algorithms are used to determine the "binding threshold" of peptides to select those with scores that give them a high probability of binding at a certain affinity and will be immunogenic. The algorithms are based either on the effects on MHC binding of a particular amino acid at a particular position, the effects on antibody binding of a particular amino acid at a particular position, or the effects on binding of a particular substitution in a motif-containing peptide. Within the context of an immunogenic peptide, a "conserved residue" is one which appears in a significantly higher frequency than would be expected by random distribution at a particular position in a peptide. In one aspect, a conserved residue is one where the MHC structure may provide a contact point with the immunogenic peptide.

Immunogenic peptides can also be identified by measuring their binding to a specific MHC protein and by their ability to stimulate CD4 and/or CD8 when presented in the context of the MHC protein. In one example, an immunogenic "Mtb peptide" is a series of contiguous amino acid residues from the Mtb protein generally between 9 and 20 amino acids in length, such as about 8 to 11 residues in length.

Generally, immunogenic Mtb polypeptides can be used to induce an immune response in a subject, such as a B cell response or a T cell response. In one example, an immunogenic Mtb polypeptide, when bound to a MHC Class I molecule, activates CD8⁺ T cells, such as cytotoxic T lymphocytes (CTLs) against Mtb. Induction of CTLs using synthetic peptides and CTL cytotoxicity assays are known in the art (see U.S. Pat. No. 5,662,907). In one example, an immunogenic peptide includes an allele-specific motif or other sequence such that the peptide will bind an MHC molecule and induce a CD8⁺ response against the antigen from which the immunogenic peptide is derived. A CD8⁺ T cell that specifically recognizes an Mtb polypeptide is activated, proliferates, and/or secretes cytokines in response to that specific polypeptide, and not to other, non-related polypeptides.

Interferon gamma (IFN-γ): IFN-γ is a dimeric protein with subunits of 146 amino acids. The protein is glycosylated at two sites, and the pI is 8.3-8.5. IFN-γ is synthesized as a precursor protein of 166 amino acids including a secretory signal sequence of 23 amino acids. Two molecular forms of the biologically active protein of 20 and 25 kDa have been described. Both of them are glycosylated at position 25. The 25 kDa form is also glycosylated at position 97. The observed differences of natural IFN-γ with respect to molecular mass and charge are due to variable glycosylation patterns. 40-60 kDa forms observed under non-denaturing conditions are dimers and tetramers of IFN-γ. The human gene has a length of approximately 6 kb. It contains four exons and maps to chromosome 12q24.1.

IFN-γ can be detected by sensitive immunoassays, such as an ELISA test that allows detection of individual cells producing IFN-γ. Minute amounts of IFN-γ can be detected indirectly by measuring IFN-induced proteins such as Mx protein. The induction of the synthesis of IP-10 has also been used to measure IFN-γ concentrations. In addition, bioassays can be used to detect IFN-γ, such as an assay that employs induction of indoleamine 2,3-dioxygenase activity in 2D9 cells. The production of IFN-γ can be used to assess T cell activation, such as activation of a T cell by a *Mycobacterium* antigen.

Isolated: An "isolated" biological component (such as a nucleic acid molecule, protein, or organelle) has been substantially separated or purified away from other biological components in the cell of the organism in which the component naturally occurs, such as other chromosomal and extra-chromosomal DNA and RNA, proteins, and organelles. Nucleic acids and proteins that have been "isolated" include nucleic acids and proteins purified by standard purification methods. The term also embraces nucleic acids and proteins prepared by recombinant expression in a host cell, as well as chemically synthesized nucleic acids or proteins, or fragments thereof.

Label: A detectable compound or composition that is conjugated directly or indirectly to another molecule to facilitate detection of that molecule. Specific, non-limiting examples of labels include fluorescent tags, enzymatic linkages, and radioactive isotopes.

Linker sequence: A linker sequence is an amino acid sequence that covalently links two polypeptide domains. Linker sequences can be included in the between the Mtb epitopes disclosed herein to provide rotational freedom to the linked polypeptide domains and thereby to promote proper domain folding and presentation to the MHC. By way of example, in a recombinant polypeptide comprising two Mtb domains, linker sequences can be provided between them, such as a polypeptide comprising Mtb polypeptide-linker-Mtb polypeptide. Linker sequences, which are generally between 2 and 25 amino acids in length, are well known in the art and include, but are not limited to, the glycine(4)-serine spacer (GGGGS×3) described by Chaudhary et al., Nature 339:394-397, 1989.

Lymphocytes: A type of white blood cell that is involved in the immune defenses of the body. There are two main types of lymphocytes: B cells and T cells.

Mycobacteria: A genus of aerobic intracellular bacterial organisms. Upon invasion of a host, these organisms survive within endosomal compartments of monocytes and macrophages. Human mycobacterial diseases include tuberculosis (caused by *M. tuberculosis*)*,* Leprosy (caused by *M. leprae*), Bairnsdale ulcers (caused by *M. ulcerans*), and other infections that can be caused by *M. marinum, M. kansasii, M. scrofulaceum, M. szulgai, M. xenopi, M. fortuitum, M. haemophilum, M. chelonei,* and *M. intracelluare. Mycobacterium* strains that were previously considered to be nonpathogenic (such as *M*. *avium*) are also now known to be major killers of immunosuppressed AIDS patients.

The major response to mycobacteria involves cell mediated hypersensitivity (delayed type hypersensitivity (DTH)) reactions with T cells and macrophages playing major roles in the intracellular killing and walling off (or containing) of the organism (granuloma formation). A major T cell response involves CD4⁺ lymphocytes that recognize mycobacterial heat shock proteins and immunodominant antigens.

Operably linked: A first nucleic acid sequence is operably linked with a second nucleic acid sequence when the first nucleic acid sequence is placed in a functional relationship with the second nucleic acid sequence. For instance, a promoter is operably linked to a coding sequence if the promoter affects the transcription or expression of the coding sequence. Generally, operably linked DNA sequences are contiguous and, where necessary to join two protein coding regions, the open reading frames are aligned.

ORF (open reading frame): A series of nucleotide triplets (codons) coding for amino acids without any termination codons. These sequences are usually translatable into a polypeptide.

Peptide Modifications: *Mycobacterium* polypeptides include synthetic aspects of peptides described herein. In addition, analogues (non-peptide organic molecules), derivatives (chemically functionalized peptide molecules obtained starting with the disclosed peptide sequences) and variants (homologs) of these proteins can be utilized in the methods described herein. Each polypeptide of the invention is comprised of a sequence of amino acids, which may be either L- and/or D-amino acids, naturally occurring and otherwise.

Peptides may be modified by a variety of chemical techniques to produce derivatives having essentially the same activity as the unmodified peptides, and optionally having other desirable properties. For example, carboxylic acid groups of the protein, whether carboxyl-terminal or side chain, may be provided in the form of a salt of a pharmaceutically-acceptable cation or esterified to form a C₁-C₁₆ester, or converted to an amide of formula NR₁R₂ wherein R₁ and R₂are each independently H or C₁-C₁₆ alkyl, or combined to form a heterocyclic ring, such as a 5- or 6-membered ring. Amino groups of the peptide, whether amino-terminal or side chain, may be in the form of a pharmaceutically-acceptable acid addition salt, such as the HCl, HBr, acetic, benzoic, toluene sulfonic, maleic, tartaric and other organic salts, or may be modified to C₁-C₁₆ alkyl or dialkyl amino or further converted to an amide.

Hydroxyl groups of the peptide side chains may be converted to C₁-C₁₆alkoxy or to a C₁-C₁₆ ester using well-recognized techniques. Phenyl and phenolic rings of the peptide side chains may be substituted with one or more halogen atoms, such as fluorine, chlorine, bromine or iodine, or with C₁-C₁₆ alkyl, C₁-C₁₆ alkoxy, carboxylic acids and esters thereof, or amides of such carboxylic acids. Methylene groups of the peptide side chains can be extended to homologous C₂-C₄ alkylenes. Thiols can be protected with any one of a number of well-recognized protecting groups, such as acetamide groups. Those skilled in the art will also recognize methods for introducing cyclic structures into the peptides of this invention to select and provide conformational constraints to the structure that result in enhanced stability.

Peptidomimetic and organomimetic aspects are envisioned, whereby the three-dimensional arrangement of the chemical constituents of such peptido- and organomimetics mimic the three-dimensional arrangement of the peptide backbone and component amino acid side chains, resulting in such peptido- and organomimetics of *a Mycobacterium* polypeptide having measurable or enhanced ability to generate an immune response. For computer modeling applications, a pharmacophore is an idealized, three-dimensional definition of the structural requirements for biological activity. Peptido- and organomimetics can be designed to fit each pharmacophore with current computer modeling software (using computer assisted drug design or CADD). See Walters, "Computer-Assisted Modeling of Drugs," in Klegerman & Groves, eds., 1993, Pharmaceutical Biotechnology, Interpharm Press, Buffalo Grove, Ill., pp. 165-174 and Principles of Pharmacology Munson (ed.) 1995, Ch. 102, for descriptions of techniques used in CADD. Also included are mimetics prepared using such techniques.

Polynucleotide: A linear nucleotide sequence, including sequences of greater than 100 nucleotide bases in length.

Polypeptide: Any chain of amino acids, regardless of length or post-translational modification (e.g., glycosylation or phosphorylation). A "peptide" is a chain of amino acids that is less than 100 amino acids in length. In one aspect, a "peptide" is a portion of a polypeptide, such as about 8-11, 9-12, or about 10, 20, 30, 40, 50, or 100 contiguous amino acids of a polypeptide that is greater than 100 amino acids in length.

Probes and primers: Nucleic acid probes and primers may readily be prepared based on the nucleic acids provided by this invention. A probe comprises an isolated nucleic acid attached to a detectable label or reporter molecule. Typical labels include radioactive isotopes, ligands, chemiluminescent agents, and enzymes. Methods for labeling and guidance in the choice of labels appropriate for various purposes are discussed, e.g., in Sambrook et al. (1989) and Ausubel et al. (1987).

Primers are short nucleic acids, preferably DNA oligonucleotides 15 nucleotides or more in length. Primers may be annealed to a complementary target DNA strand by nucleic acid hybridization to form a hybrid between the primer and the target DNA strand, and then extended along the target DNA strand by a DNA polymerase enzyme. Primer pairs can be used for amplification of a nucleic acid sequence, e.g., by the polymerase chain reaction (PCR) or other nucleic-acid amplification methods known in the art.

Methods for preparing and using probes and primers are described, for example, in Molecular Cloning: A Laboratory Manual, 2nd ed., vol. 1-3, ed. Sambrook et al., Cold Spring Harbor Laboratory Press, Cold Spring Harbor, N.Y., 1989, and Current Protocols in Molecular Biology, ed. Ausubel et al., Greene Publishing and Wiley-Interscience, New York, 1987 (with periodic updates). PCR primer pairs can be derived from a known sequence, for example, by using computer programs intended for that purpose such as Primer3 (Version 0.4.0, Whitehead Institute for Biomedical Research, Cambridge, Mass.).

Promoter: A promoter is an array of nucleic acid control sequences which direct transcription of a nucleic acid. A promoter includes necessary nucleic acid sequences near the start site of transcription, such as, in the case of a polymerase II type promoter, a TATA element. A promoter also optionally includes distal enhancer or repressor elements which can be located as much as several thousand base pairs from the start site of transcription. The promoter can be a constitutive or an inducible promoter. A specific, non-limiting example of a promoter is the HCMV IE promoter.

Purified: The term purified does not require absolute purity; rather, it is intended as a relative term. Thus, for example, a purified antigen preparation is one in which the antigen is purer than the protein in its originating environment within a cell. A preparation of an antigen is typically purified such that the antigen represents at least 50% of the total protein content of the preparation. However, more highly purified preparations may be required for certain applications. For example, for such applications, preparations in which the antigen comprises at least 75% or at least 90% of the total protein content may be employed.

Recombinant: A recombinant nucleic acid or polypeptide is one that has a sequence that is not naturally occurring or has a sequence that is made by an artificial combination of two or more otherwise separated segments of sequence. This artificial combination is often accomplished by chemical synthesis or, more commonly, by the artificial manipulation of isolated segments of nucleic acids, e.g., by genetic engineering techniques.

Sequence identity: The similarity between amino acid sequences is expressed in terms of the similarity between the sequences, otherwise referred to as sequence identity. Sequence identity is frequently measured in terms of percentage identity (or similarity or homology); the higher the percentage, the more similar the two sequences are. Variants of antigen polypeptides will possess a relatively high degree of sequence identity when aligned using standard methods. Percent identity is calculated by multiplying the number of matches in a pair of proteins, peptides, or nucleic acids by 100 and dividing by the length of the aligned region, including gaps. Identity scoring only counts perfect matches, and does not consider the degree of similarity of amino acids to one another. Note that only internal gaps are included in the length, not gaps at the sequence ends.

Methods of alignment of sequences for comparison are well known in the art. A detailed consideration of sequence alignment methods and homology calculations was published in 1994. The NCBI Basic Local Alignment Search Tool (BLAST) is available from several sources, including the National Center for Biotechnology Information (NCBI, Bethesda, Md.) and on the Internet, for use in connection with the sequence analysis programs blastp, blastn, blastx, tblastn and tblastx. It can be accessed at the NCBI website. A description of how to determine sequence identity using this program is available at the NCBI website, as are the default parameters.

Variants of antigenic polypeptides, such as *a Mycobacterium* polypeptides, are typically characterized by possession of at least 80% sequence identity counted over the full length alignment with the amino acid sequence of a native antigen sequence using the NCBI Blast 2.0, gapped blastp set to default parameters. Proteins with even greater similarity to the reference sequences will show increasing percentage identities when assessed by this method, such as at least 85%, at least 87%, at least 90%, at least 92%, at least 93%, at least 94%, at least 95% , at least 96%, at least 97%, at least 98%, or at least 99% sequence identity. When less than the entire sequence is being compared for sequence identity, variants will typically possess at least 80% sequence identity over short windows of 10-20 amino acids, and may possess sequence identities of at least 85% or at least 90% or 95% depending on their similarity to the reference sequence. Methods for determining sequence identity over such short windows are described at the NCBI website.

Transduced and Transformed: A virus or vector "transduces" a cell when it transfers nucleic acid into the cell. A cell is "transformed" by a nucleic acid transduced into the cell when the DNA becomes stably replicated by the cell, either by incorporation of the nucleic acid into the cellular genome, or by episomal replication. As used herein, the term transformation encompasses all techniques by which a nucleic acid molecule might be introduced into such a cell, including transfection with viral vectors, transformation with plasmid vectors, and introduction of naked DNA by electroporation, lipofection, and particle gun acceleration.

Tuberculosis (TB): A disease that is generally caused by *Mycobacterium tuberculosis* that usually infects the lungs. However, other "atypical" mycobacteria such as *M. kansasii* may produce a similar clinical and pathologic appearance of disease.

Transmission of *M. tuberculosis* occurs by the airborne route in confined areas with poor ventilation. In more than 90% of cases, following infection with *M. tuberculosis,* the immune system prevents development of disease from *M. tuberculosis,* often called, active tuberculosis. However, not all of the *M. tuberculosis* is killed, and thus tiny, hard capsules are formed. "Primary tuberculosis" is seen as disease that develops following an initial infection, usually in children. The initial focus of infection is a small subpleural granuloma accompanied by granulomatous hilar lymph node infection. Together, these make up the Ghon complex. In nearly all cases, these granulomas resolve and there is no further spread of the infection. "Secondary tuberculosis" is seen mostly in adults as a reactivation of previous infection (or reinfection), particularly when health status declines. The granulomatous inflammation is much more florid and widespread. Typically, the upper lung lobes are most affected, and cavitation can occur. Dissemination of tuberculosis outside of the lungs can lead to the appearance of a number of uncommon findings with characteristic patterns that include skeletal tuberculosis, genital tract tuberculosis, urinary tract tuberculosis, central nervous system (CNS) tuberculosis, gastrointestinal tuberculosis, adrenal tuberculosis, scrofula, and cardiac tuberculosis. "Latent" tuberculosis is an Mtb infection in an individual that can be detected by a diagnostic assay, such as, but not limited to a tuberculin skin test (TST) or the methods provided herein, wherein the infection does not produce symptoms in that individual. "Active" tuberculosis is a symptomatic Mtb infection in a subject. Cases of active tuberculosis as caused by Mtb infection may be identified using the methods provided herein.

Treatment of a subject having an immune response to *Mycobacterium tuberculosis* is also disclosed. A subject identified as having an immune response toM. *tuberculosis* may be treated to inhibit development of a disease, such as tuberculosis or to ameliorate a sign or symptom of a disease or pathological condition related to tuberculosis.

Microscopically, the inflammation produced with TB infection is granulomatous, with epithelioid macrophages and Langhans giant cells along with lymphocytes, plasma cells, maybe a few polymorphonuclear cells, fibroblasts with collagen, and characteristic caseous necrosis in the center. The inflammatory response is mediated by a type IV hypersensitivity reaction, and skin testing is based on this reaction. In some examples, tuberculosis can be diagnosed by a skin test, an acid fast stain, an auramine stain, or a combination thereof. The most common specimen screened is sputum, but the histologic stains can also be performed on tissues or other body fluids.

TB is a frequent complication of HIV infection. TB infection in subjects infected with a human immunodeficiency virus (HIV) can spread readily and progress rapidly to active disease. Specific symptoms of lung disease due to Mtb infection include chronic cough and spitting blood. Other symptoms of TB disease include fatigue, loss of appetite, weight loss, fever and drenching night sweats.

Vector: A nucleic acid molecule as introduced into a host cell, thereby producing a transformed host cell. A vector may include nucleic acid sequences that permit it to replicate in a host cell, such as an origin of replication. A vector may also include one or more selectable marker gene and other genetic elements known in the art. Vectors include plasmid vectors, including plasmids for expression in gram negative and gram positive bacterial cell. Exemplary vectors include those for expression in *E. coli* and *Salmonella.* Vectors also include viral vectors, such as, but not limited to, retrovirus, orthopox, avipox, fowlpox, capripox, suipox, adenovirus, herpes virus, alpha virus, baculovirus, Sindbis virus, vaccinia virus, and poliovirus vectors. Vectors also include vectors for expression in yeast cells.

### METHODS

### Detection of T Cells that Recognize a Mtb Peptide

The present disclosure provides methods for detecting an immune response to *Mycobacterium tuberculosis* (Mtb) in a subject. An immune response to *Mycobacterium tuberculosis* can be detected based on the presence of T cells in a biological sample, which T cells specifically react with a Mtb peptide disclosed herein (e.g., SEQ ID NOs: 1-15). In all aspects of the invention, at least one peptide is used that is 8 to 18 amino acids long and comprises an amino acid sequence having at least 70% identity to SEQ ID NO: 3, although where explicitly specified in the claims, the length of the peptide is 9 to 18 amino acids. Thus, in any aspects of the invention wherein the at least one peptide is a single peptide, it is a single peptide comprising or consisting of an amino acid sequence having at least 70% identity to the amino acid sequence set forth in SEQ ID NO: 3. In any aspects wherein the at least one peptide is not a single peptide, at least one of the peptides is 8 to 18 amino acids long (or 9 to 18 acids long, where specified in the claims) and comprises or consists of an amino acid sequence having at least 70% identity to SEQ ID NO: 3. The disclosure herein regarding characteristics of the at least one peptide should be read with this in mind. The presence of T cells that specifically react with a Mtb peptide disclosed herein is indicative *of Mycobacterium tuberculosis* infection. The infection may be latent or active.

The method may include contacting a biological sample comprising T cells from the subject with at least one peptide and determining the presence of T cells in the biological sample that specifically recognize the amino acid sequence of the at least one peptide, wherein the presence of T cells that specifically recognize the amino acid sequence of the at least one peptide indicates that the subject has an immune response to *Mycobacterium tuberculosis.* The at least one peptide may comprise an immunogenic amino acid sequence derived from the amino acid sequence of a polypeptide produced by any *Mycobacterium tuberculosis* strain. The amino acid sequence of the at least one peptide may correspond to any portion of the full-length Mtb polypeptide from which it is derived, where the portion is sufficient for activating T cells. The polypeptide from which the at least one peptide is derived may be ctpF (Accession No: ANZ82682), PPE15 (Accession No: AOE35388), PPE68 (Accession No: AOE38353), PE3 (Accession No: AOE34479), irtA (Accession No: ANZ82019), Rv0219 (Accession No: CCP42947), PE12 (Accession No: AOE35536), esxJ (Accession No: CCE36570), esxG (Accession No: CCE35829), PE_PGRS50 (Accession No: CCP46166), or PPE51 (Accession No: AOE37558).

The length of the at least one peptide may vary. The at least one peptide may range in length from 8 to 12 amino acids, from 8 to 18 amino acids, from 8 to 20 amino acids, from 8 to 25 amino acids, 9 to 12 amino acids, from 9 to 18 amino acids, from 9 to 20 amino acids, or from 9 to 25 amino acids. In some cases, the at least one peptide may range in length from 15 amino acids to 886 amino acids, from 15 to 18 amino acids, from 11 to 18 amino acids, from 13 to 466 amino acids, from 16 to 315 amino acids, from 12 to 16 amino acids, from 11 to 33 amino acids, from 13 to 16 amino acids, from 13 to 21 amino acids, from 9 to 16 amino acids, or from 8 to 16 amino acids. In some cases, the at least one peptide includes an additional 1 to 5 amino acids that extends the length of the at least one peptide at the N terminus, where the additional amino acids are derived from the corresponding amino acid sequence of the *Mycobacterium* polypeptide from which the at least one peptide is derived. In some cases, the at least one peptide includes an additional 1 to 5 amino acids that extends the length of the at least one peptide at the C terminus, where the additional amino acids are derived from the corresponding amino acid sequence of the *Mycobacterium* polypeptide from which the at least one peptide is derived.

The at least one peptide used in the method disclosed herein may have a length of 9 to 18 amino acids. The at least one peptide may have 8 to 18 amino acids long sequence (e.g., from 9 to 12 amino acids) at least 80%, at least 85%, at least 90%, at least 95%, at least 96%, at least 97%, at least 98% or at least 99% identical to the amino acid sequence set forth in SEQ ID NO:1, SEQ ID NO:2, SEQ ID NO:6, SEQ ID NO:7, SEQ ID NO:11, SEQ ID NO: 14, SEQ ID NO: 4, SEQ ID NO: 15, SEQ ID NO: 8, SEQ ID NO: 3, SEQ ID NO: 13, SEQ ID NO: 12, SEQ ID NO: 5, or SEQ ID NO: 10.

In certain aspects, the at least one peptide is a single peptide having a length of 8 to 18 amino acids (e.g., from 9 to 12 amino acids) and comprising an amino acid sequence having at least 70%, at least 75%, at least 85%, at least 90%, at least 95%, at least 96%, at least 97%, at least 98% or at least 99% identity to the amino acid sequence set forth in SEQ ID NO: 3. In some cases, the at least one peptide is at least two peptides, the (further) peptide having a length of 8 to 18 amino acids and comprising an amino acid sequence having at least 60%, at least 70%, at least 75%, at least 85%, at least 90%, at least 95%, at least 96%, at least 97%, at least 98% or at least 99% identity to the amino acid sequence set forth in SEQ ID NO:1, SEQ ID NO:2, SEQ ID NO:6, SEQ ID NO:7, SEQ ID NO:11, SEQ ID NO: 14, SEQ ID NO: 4, SEQ ID NO: 15, SEQ ID NO: 8, SEQ ID NO: 3, SEQ ID NO: 13, SEQ ID NO: 12, SEQ ID NO: 5, or SEQ ID NO: 10. In some cases, the at least one peptide is at least three peptides having 9 to 18 amino acids long sequences, the (further) peptides each independently comprising an amino acid sequence having at least 60%, at least 70%, at least 75%, at least 85%, at least 90%, at least 95%, at least 96%, at least 97%, at least 98% or at least 99% identity to the amino acid sequence set forth in SEQ ID NO:1, SEQ ID NO:2, SEQ ID NO:6, SEQ ID NO:7, SEQ ID NO:11, SEQ ID NO: 14, SEQ ID NO: 4, SEQ ID NO: 15, SEQ ID NO: 8, SEQ ID NO: 3, SEQ ID NO: 13, SEQ ID NO: 12, SEQ ID NO: 5, or SEQ ID NO: 10. In some cases, the at least one peptide is at least four peptides having 9 to 18 amino acids long sequences, the (further) peptides each independently comprising an amino acid sequence having at least 60%, at least 70%, at least 75%, at least 85%, at least 90%, at least 95%, at least 96%, at least 97%, at least 98% or at least 99% identity to the amino acid sequence set forth in SEQ ID NO:1, SEQ ID NO:2, SEQ ID NO:6, SEQ ID NO:7, SEQ ID NO:11, SEQ ID NO: 14, SEQ ID NO: 4, SEQ ID NO: 15, SEQ ID NO: 8, SEQ ID NO: 3, SEQ ID NO: 13, SEQ ID NO: 12, SEQ ID NO: 5, or SEQ ID NO: 10. In some cases, the at least one peptide is at least five peptides having 9 to 18 amino acids long sequences, the (further) peptides each independently comprising an amino acid sequence having at least 60%, at least 70%, at least 75%, at least 85%, at least 90%, at least 95%, at least 96%, at least 97%, at least 98% or at least 99% identity to the amino acid sequence set forth in SEQ ID NO:1, SEQ ID NO:2, SEQ ID NO:6, SEQ ID NO:7, SEQ ID NO:11, SEQ ID NO: 14, SEQ ID NO: 4, SEQ ID NO: 15, SEQ ID NO: 8, SEQ ID NO: 3, SEQ ID NO: 13, SEQ ID NO: 12, SEQ ID NO: 5, or SEQ ID NO: 10. In some cases, the at least one peptide is at least six peptides having 9 to 18 amino acids long sequences, the (further) peptides each independently comprising an amino acid sequence having at least 60%, at least 70%, at least 75%, at least 85%, at least 90%, at least 95%, at least 96%, at least 97%, at least 98% or at least 99% identity to the amino acid sequence set forth in SEQ ID NO:1, SEQ ID NO:2, SEQ ID NO:6, SEQ ID NO:7, SEQ ID NO:11, SEQ ID NO: 14, SEQ ID NO: 4, SEQ ID NO: 15, SEQ ID NO: 8, SEQ ID NO: 3, SEQ ID NO: 13, SEQ ID NO: 12, SEQ ID NO: 5, or SEQ ID NO: 10. In some cases, the at least one peptide is at least seven peptides, at least eight peptides, at least nine peptides, at least ten peptides, at least eleven peptides, at least twelve peptides, at least thirteen peptides, or at least fourteen peptides having 9 to 18 amino acids long sequences, the (further) peptides each independently comprising an amino acid sequence having at least 60%, at least 70%, at least 75%, at least 85%, at least 90%, at least 95%, at least 96%, at least 97%, at least 98% or at least 99% identity to the amino acid sequence set forth in SEQ ID NO:1, SEQ ID NO:2, SEQ ID NO:6, SEQ ID NO:7, SEQ ID NO:11, SEQ ID NO: 14, SEQ ID NO: 4, SEQ ID NO: 15, SEQ ID NO: 8, SEQ ID NO: 3, SEQ ID NO: 13, SEQ ID NO: 12, SEQ ID NO: 5, SEQ ID NO: 10. In certain aspects, the method may further include contacting the biological sample comprising T cells from the subject with a peptide comprising the amino acid sequence of SEQ ID NO:9 and determining the presence of T cells in the biological sample that specifically recognize the amino acid sequence of the peptide.

In certain aspects, the at least one peptide comprises an amino acid sequence set forth in: RPELPVRNYIRIPAL (SEQ ID NO:1); LHEARTAALNLFVVV (SEQ ID NO: 2); YQTYMYTGQYDGY (SEQ ID NO: 6); IAPHFVRVRMVSPTLF (SEQ ID NO: 7); RRRCRPWLHRR (SEQ ID NO: 11); YVGLYLPFLDMSF (SEQ ID NO: 14); GPRYGFKPTVM (SEQ ID NO: 4); NEIRALMAL (SEQ ID NO: 15); YYLGEPIVQAVL (SEQ ID NO: 8); WMGPASMAMVAAAQP (SEQ ID NO: 3); RQFHHPLIYVLLV (SEQ ID NO: 13); TQALQALTIPSF (SEQ ID NO: 12); TLFEKLEPMASIL (SEQ ID NO: 5); or AMSAQAFHQGESSAAF (SEQ ID NO: 10). In certain aspects, the at least one peptide is at least two peptides, at least three peptides, at least four peptides, at least five peptides, at least six peptides, at least seven peptides, at least eight peptides, at least nine peptides, at least ten peptides, at least eleven peptides, at least twelve peptides, at least thirteen peptides, at least fourteen peptides, or fifteen peptides, the (further) peptides each independently comprising the amino acid sequence set forth in SEQ ID NO:1, SEQ ID NO:2, SEQ ID NO:6, SEQ ID NO:7, SEQ ID NO:11, SEQ ID NO: 14, SEQ ID NO: 4, SEQ ID NO: 15, SEQ ID NO: 8, SEQ ID NO: 3, SEQ ID NO: 13, SEQ ID NO: 12, SEQ ID NO: 5, SEQ ID NO: 10, or SEQ ID NO:9.

In certain aspects, the at least one peptide consists essentially of or consists of the amino acid sequence set forth in SEQ ID NO:1, SEQ ID NO:2, SEQ ID NO:6, SEQ ID NO:7, SEQ ID NO:11, SEQ ID NO: 14, SEQ ID NO: 4, SEQ ID NO: 15, SEQ ID NO: 8, SEQ ID NO: 3, SEQ ID NO: 13, SEQ ID NO: 12, SEQ ID NO: 5, or SEQ ID NO: 10. In some cases, the at least one peptide is at least two peptides, at least three peptides, at least four peptides, at least five peptides, at least six peptides, at least seven peptides, at least eight peptides, at least nine peptides, at least ten peptides, at least eleven peptides, at least twelve peptides, at least thirteen peptides, or fourteen peptides, the (further) peptide(s) each independently consisting essentially of or consisting of the amino acid sequence set forth in SEQ ID NO:1, SEQ ID NO:2, SEQ ID NO:6, SEQ ID NO:7, SEQ ID NO:11, SEQ ID NO: 14, SEQ ID NO: 4, SEQ ID NO: 15, SEQ ID NO: 8, SEQ ID NO: 3, SEQ ID NO: 13, SEQ ID NO: 12, SEQ ID NO: 5, or SEQ ID NO: 10.

In some cases, the at least ten peptides comprise, consist essentially of, or consist of the amino acid sequences set forth in SEQ ID NO:1, SEQ ID NO:2, SEQ ID NO:6, SEQ ID NO:7, SEQ ID NO:11, SEQ ID NO:14, SEQ ID NO:4, SEQ ID NO:15, SEQ ID NO:8, and SEQ ID NO:3. In some cases, the at least eleven peptides comprise, consist essentially of, or consist of the amino acid sequences set forth in SEQ ID NO:1, SEQ ID NO:2, SEQ ID NO:6, SEQ ID NO:7, SEQ ID NO:11, SEQ ID NO:14, SEQ ID NO:4, SEQ ID NO:15, SEQ ID NO:8, SEQ ID NO:3, and SEQ ID NO:13. In some cases, the at least twelve peptides comprise, consist essentially of, or consist of the amino acid sequences set forth in SEQ ID NO:1, SEQ ID NO:2, SEQ ID NO:6, SEQ ID NO:7, SEQ ID NO:11, SEQ ID NO:14, SEQ ID NO:4, SEQ ID NO:15, SEQ ID NO:8, SEQ ID NO:3, SEQ ID NO:13, and SEQ ID NO:12. In some cases, the at least thirteen peptides comprise, consist essentially of, or consist of the amino acid sequences set forth in SEQ ID NO:1, SEQ ID NO:2, SEQ ID NO:6, SEQ ID NO:7, SEQ ID NO:11, SEQ ID NO:14, SEQ ID NO:4, SEQ ID NO:15, SEQ ID NO:8, SEQ ID NO:3, SEQ ID NO:13, SEQ ID NO:12, and SEQ ID NO:5. In some cases, the at least fourteen peptides comprise, consist essentially of, or consist of the amino acid sequences set forth in SEQ ID NO:1, SEQ ID NO:2, SEQ ID NO:6, SEQ ID NO:7, SEQ ID NO:11, SEQ ID NO:14, SEQ ID NO:4, SEQ ID NO:15, SEQ ID NO:8, SEQ ID NO:3, SEQ ID NO:13, SEQ ID NO:12, SEQ ID NO:5, and SEQ ID NO:10. In some cases, the fifteen peptides comprise, consist essentially of, or consist of the amino acid sequences set forth in SEQ ID NO:1, SEQ ID NO:2, SEQ ID NO:6, SEQ ID NO:7, SEQ ID NO:11, SEQ ID NO:14, SEQ ID NO:4, SEQ ID NO:15, SEQ ID NO:8, SEQ ID NO:3, SEQ ID NO:13, SEQ ID NO:12, SEQ ID NO:5, SEQ ID NO:10, and SEQ ID NO:9.

A biological sample comprising T cells may be obtained from a subject of interest. Suitable biological samples include, but are not limited to, blood samples, isolated peripheral blood mononuclear cells, isolated mononuclear cells, sputum, saliva, cerebrospinal fluid or samples of isolated T cells (such as CD8⁺ T cells and/or CD4⁺ T cells), lymph node tissue, lung tissue, or other tissue sample. In some cases, the biological sample includes antigen presenting cells. In one example, the sample is incubated with the at least one peptide, as disclosed herein, a cell expressing the peptide, or an APC that expresses the peptide. The presence or absence of specific activation of the T cells is detected.

The CD8⁺ T cells and/or CD4⁺ T cells which recognize the peptide in the detection method disclosed herein have generally been presensitized in vivo to Mtb antigens if the subject has been exposed to *M. tuberculosis,* for example, due to a latent or active Mtb infection. In several aspects, these antigen-experienced T cells are generally present in the peripheral blood of a subject which has been exposed to the antigen at a frequency of 1 in 10⁶ to 1 in 10³ peripheral blood mononuclear cells (PBMCs).

In one example, the sample comprises isolated T cells. For example, T cells can be isolated from a subject of interest by routine techniques (such as by Ficoll/Hypaque density gradient centrifugation of peripheral blood lymphocytes, or by fluorescence activated cell sorting). In one aspect, the T cells used in the assay are in the form of unprocessed or diluted samples, or are freshly isolated T cells (such as in the form of freshly isolated mononuclear cells (MCs) or PBMCs which are used directly ex vivo, such that they are not cultured before being used in the method. However, the T cells can be cultured before use, for example in the presence of one or more of the peptides, and generally also exogenous growth promoting cytokines. During culturing, the peptides are typically presented on the surface of cells such as APCs. Preculturing of the T cells may lead to an increase in the sensitivity of the method. Thus, the T cells can be converted into cell lines, such as short term cell lines.

In several aspects, the T cells are incubated *in vitro* for two to nine days, such as about four days, at 37° C with an Mtb peptide or fragment thereof that binds MHC. In several examples, the Mtb peptide or fragment thereof that binds MHC is included (at a concentration of, for example, about 5 to about 25 µg/ml, such as about 5, about 10, about 15, or about 20 µg/ml). In several examples, another aliquot of a T cell sample can be incubated in the absence of the Mtb polypeptide as a control.

In one aspect, MCs are separated from the sample. The MCs include the T cells and APCs. Thus, in the method the APCs present in the separated MCs can present the peptide to the T cells. In another aspect, only T cells, such as only CD8⁺ T cells, only CD4⁺ T cells, or only CD3⁺ T cells, can be purified from the biological sample obtained from the subject.

The APC used in the disclosed methods may be any cell which has MHC class I molecules on its surface. In some cases, the APC is a specialized antigen presenting cell, such as a B cell, dendritic cell or macrophage. The APC used in the method may be from the same host as the T cell. Generally, the APC is capable of presenting the peptide to a T cell. The APC can be a freshly isolated *ex vivo* cell or a cultured cell such as a cell from of a cell line.

T cells derived from a sample from a subject of interest can be placed into an assay with at least one of the Mtb peptides disclosed herein (e.g., a pool of the Mtb peptides) or the T cells can be placed into separate assays each of which contain one or more of the peptides. In one aspect, one or more of the peptides with an amino acid sequence set forth as SEQ ID NOs: 1-15, or a fragment of one or more Mtb polypeptides that bind MHC, is utilized, as described in detail above. Two or more of any of the Mtb peptides disclosed herein can be used for simultaneous, separate or sequential detection of T cells that recognize these peptides. Additional combinations of any of the Mtb peptides disclosed herein can be utilized, as described above.

In one aspect the one or more peptide(s) is (are) provided to the presenting cell in the absence of the T cell. This cell is then provided to T cells isolated from the subject, typically after being allowed to present the peptide on its surface.

The duration for which the peptide is contacted with the cells will vary depending on the method used for determining recognition of the peptide. Typically, 10⁵ to 10⁷, such as 5×10⁵ to 10⁶ PBMCs are added to each assay. In the case where the at least one peptide is added directly to the assay, its concentration is typically from 10⁻¹ to 10³ µg/ml, such as about 0.5 to about 50 µg/ml or about 1 to about 10 µg/ml. The length of time for which the T cells from a subject are incubated with the at least one peptide can be from about 4 to about 24 hours, such as from about 6 to about 16 hours, or for about 12 hours.

The method further includes determining the presence of T cells in the biological sample that specifically recognize the amino acid sequence of the at least one peptide as provided herein. Specific recognition of the at least one peptide by the T cells can be detected by measuring the binding of the peptide to the T cells or by detecting a change in the state of the T cells in the presence of the peptide. The change in state is generally caused by antigen specific functional activity of the T cell after the T cell receptor binds the peptide. The peptide may be bound to an MHC class I molecule, which may be present on the surface of a PBMC or an APC. T cell activation can be detected by any means known to one of skill in the art.

In certain aspects, T cells which bind the at least one peptide can be sorted based on this binding, for example using a fluorescence activated cell sorting (FACS) technique. The detection of the presence of T cells which recognize the peptide will be deemed to occur if the frequency of cells sorted using the peptide is above a control value.

In certain aspects, CD8⁺ T cell activation is detected by evaluating cytolytic activity. In another example, CD8⁺ T cell activation and/or CD4⁺ T cell activation is detected by proliferation. A level of proliferation that is at least two-fold greater and/or a level of cytolytic activity that is at least 20% greater than in uninfected subjects indicates the presence of a Mycobacterium tuberculosis infection in the subject of interest.

The change in state of the T cell detected may also be the start of or an increase in secretion of a substance from the T cell, such as a cytokine, such as interferon (IFN)-γ, IL-2, IP-10 and/or TNF (e.g., TNF-α). In one example, the substance can be detected by allowing it to bind to a specific binding agent and then measuring the presence of the specific binding agent/substance complex. The specific binding agent is typically an antibody, such as polyclonal or monoclonal antibodies that binds the substance, such as the cytokine. Antibodies to cytokines are commercially available, or can be generated using standard techniques.

In some cases, the specific binding agent, such as the antibody, is immobilized on a solid support. After the cytokine is allowed to bind, the solid support can optionally be washed to remove material which is not specifically bound to the antibody. The antibody/cytokine complex can be detected by using a second binding agent which will bind the complex, such as an antibody that is labeled (either directly or indirectly) with a detectable label. Generally, the second agent binds the substance at a site which is different from the site which binds the first agent.

In some cases, the second binding agent can be detected by a third agent which is labeled directly or indirectly by a detectable label. For example, the second agent may include biotin, allowing detection by a third agent which comprises a streptavidin and a label, such as an enzymatic, radioactive or fluorescent label.

In one aspect the detection system is an ELISPOT assay, such as the assay described in US8617821B2. In one example, IFN-γ secreted from the T cell is bound by a first IFN-γ specific antibody which is immobilized on a solid support. The bound IFN-γ is then detected using a second IFN-γ specific antibody which is labeled with a detectable label. Exemplary labeled antibodies are commercially available, such as from MABTECH^{™} (Stockholm, Sweden). An exemplary ELISPOT assay is described in the Examples section below.

In certain aspects, the detection system is an enzyme linked immunosorbent assay (ELISA). This assay can be performed by first contacting a polypeptide antigen that has been immobilized on a solid support (such as in the well of a microtiter plate) with the sample in a manner such that that antibodies present within the sample that specifically bind the polypeptide of interest bind the immobilized polypeptide. Unbound sample is then removed and a detection reagent capable of binding to the immobilized antibody-polypeptide complex is added. The amount of detection reagent that remains bound is determined using a method appropriate for the specific detection reagent. For example, the detection method can detect fluorescence or the presence of an enzymatic activity.

In some aspects, the polypeptide is immobilized on the support; any remaining protein binding sites on the support are typically blocked. Any suitable blocking agent can be used to block the unbound protein binding sites, such as bovine serum albumin or TWEEN 20^{™}. The immobilized polypeptide is then incubated with the sample, and the antibody is allowed to bind to the antigen. The sample can be diluted with a suitable diluent, for example a buffer such as phosphate-buffered saline (PBS) prior to incubation. In general, an appropriate contact time (incubation time) is a period of time that is sufficient to detect the presence of antibody in a *Mycobacterium-*infected sample. In one specific, non-limiting example, the contact time is sufficient to achieve a level of binding that is at least 95% of that achieved at equilibrium between bound and unbound antibody. The time necessary to achieve equilibrium can be determined by assaying the level of binding that occurs over a period of time. At room temperature, an incubation time of about 30 minutes is generally sufficient.

Unbound sample can then be removed by washing the solid support with an appropriate buffer, such as PBS containing 0.1% TWEEN 20^{™}. A detection reagent can then be added to the solid support. A detection reagent can be any compound that binds to the immobilized antibody-polypeptide complex and can be detected. In several aspects, the detection reagent contains a binding agent (such as, for example, Protein A, Protein G, immunoglobulin, lectin or free antigen) conjugated to a label. Labels of use include enzymes (such as horseradish peroxidase or alkaline phosphatase), substrates, cofactors, inhibitors, dyes, radionuclides, luminescent groups, fluorescent groups and biotin. The conjugation of a binding agent to a label can be achieved using methods known in the art; conjugated binding agents are also commercially available (such as from Zymed Laboratories, San Francisco, Calif., and Pierce, Rockford, Ill.).

The detection reagent is incubated with the immobilized antibody-polypeptide complex for an amount of time sufficient to detect the bound antibody. An appropriate amount of time may generally be determined from the manufacturer's instructions or by assaying the level of binding that occurs over a period of time. Unbound detection reagent is then removed and bound detection reagent is detected using the label. For radioactive labels, scintillation counting or autoradiographic methods can be used for detection. Spectroscopic methods may be used to detect dyes, luminescent groups and fluorescent groups used as labels. Biotin can be detected using (strept)avidin coupled to a different label, such as a radioactive label, fluorescent label or an enzymatic label. Enzymatic labels can be detected by the addition of substrate (generally for a specific period of time), followed by spectroscopic or other analysis of the reaction products.

In some cases, the determining comprises measuring an amount of a cytokine inside of the CD8+ T cells. The amount of a cytokine inside of the CD8+ T cells may be measured with any suitable intracellular cytokine assay known in the art. In one aspect, the biological sample may be cultured in the presence of at least one peptide and then harvested, stained for CD4 and CD3, and washed. After washing, the cells may be fixed and permeabilized, using a Cytofix/Cytoperm kit (BD Biosciences) and then stained for cytokines, such as, e.g., IFN-γ (anti-IFN-γ-APC, 4S.B3), TNG, e.g., TNFα (anti-TNFα-FITC, MAb11), IP-10, and IL-2 (anti-IL-2-PE, MQ1-17H12).

The change in state of the T cell also may be an increase in the uptake of substances by the T cell, such as the uptake of thymidine. The change in state can also be measured by an increase in the size of the T cells, or proliferation of the T cells, or a change in cell surface markers on the T cell.

Reagents are provided herein for the detection of CD8 expressing cells (CD8⁺) that specifically bind an Mtb peptide as disclosed herein. These reagents are tetrameric MHC Class I/immunogenic TARP polypeptide complexes. These tetrameric complexes include an Mtb peptide, such as, e.g., a peptide of 9 to 18 amino acids in length, that specifically binds MHC class I.

Tetrameric MHC Class I/peptide complexes can be synthesized using methods well known in the art (Altmann et al., Science 274:94, 1996). In one specific non-limiting example, purified HLA heavy chain polypeptide and β2-microglobulin (β2m) can be synthesized by means of a prokaryotic expression system. One specific, non-limiting example of an expression system of use is the pET system (R&D Systems, Minneapolis, Minn.). The heavy chain is modified by deletion of the trans-membrane and cytosolic tail and COOH-terminal addition of a sequence containing the biotin protein ligase (Bir-A) enzymatic biotinylation site. Heavy chain polypeptide, β2m, and peptide are then refolded. The refolded product can be isolated by any means known in the art, and then biotinylated by Bir-A. A tetramer is then produced by contacting the biotinylated product with streptavidin.

In one aspect, the streptavidin is labeled. Suitable labels include, but are not limited to, enzymes, magnetic beads, colloidal magnetic beads, haptens, fluorochromes, metal compounds, radioactive compounds or drugs. The enzymes that can be conjugated to streptavidin include, but are not limited to, alkaline phosphatase, peroxidase, urease and β-galactosidase. The fluorochromes that can be conjugated to the streptavidin include, but are not limited to, fluorescein isothiocyanate, tetramethylrhodamine isothiocyanate, phycoerythrin, allophycocyanins and Texas Red. For additional fluorochromes that can be conjugated to streptavidin, see Haugland, Molecular Probes: Handbook of Fluorescent Probes and Research Chemicals (1992-1994). The metal compounds that can be conjugated to the streptavidin include, but are not limited to, ferritin, colloidal gold, and particularly, colloidal superparamagnetic beads. The haptens that can be conjugated to the streptavidin include, but are not limited to, biotin, digoxigenin, oxazalone, and nitrophenol. The radioactive compounds that can be conjugated to streptavidin are known to the art, and include but are not limited to technetium 99m (⁹⁹Tc), ¹²⁵I and amino acids comprising any radionuclides, including, but not limited to, ¹⁴C, ³H and ³⁵S. Generally, streptavidin labeled with a fluorochrome is utilized in the methods disclosed herein.

In one aspect, suspension of cells including T cells that specifically recognize an Mtb polypeptide is produced, and the cells are reacted with the tetramer in suspension. In one aspect, these reagents are used to label cells, which are then analyzed by fluorescence activated cell sorting (FACS). A machine for FACS employs a plurality of color channels, low angle and obtuse light-scattering detection channels, and impedance channels, among other more sophisticated levels of detection, to separate or sort cells. Any FACS technique can be employed as long as it is not detrimental to the detection of the desired cells. (For exemplary methods of FACS see U.S. Pat. No. 5,061,620).

### Skin Test

In another aspect, this invention provides at least one 9 to 18 amino acids long peptide comprising an amino acid sequence having at least 70% identity to SEQ ID NO: 3 for use in a method for detecting an immune response to *Mycobacterium tuberculosis* in a subject. The method includes administering into the skin of the subject an effective amount of at least one peptide, as described herein. The one or more of the peptides described above may be used to diagnose *a Mycobacterium* infection, and in particular tuberculosis, using a skin test. A "skin test" is any assay performed directly on a patient in which a delayed-type hypersensitivity (DTH) reaction (such as induration, swelling, reddening or dermatitis) is measured following administration into the skin, such as the intradermal injection of one or more peptides described above.

In some cases, the administering includes injecting the at least one peptide. Such injection can be achieved using any suitable device sufficient to contact the at least one peptide with dermal cells of the patient, such as a tuberculin syringe or 1 ml syringe. In several examples, the reaction is measured at least 48 hours after injection, such as between about 48 and about 72 hours after injection.

A DTH reaction is a cell-mediated immune response which is greater in subjects that have been exposed previously to the test antigen (the Mtb polypeptide, fragment thereof that binds MHC, or fusion protein thereof). The response can be measured visually, such as using a ruler. In several examples, a response that is greater than about 0.5 cm in diameter, such as greater than about 1.0 cm in diameter, is a positive response, and is indicative of *Mycobacterium* infection.

The Mtb peptides disclosed herein can be formulated for use in a skin test as pharmaceutical compositions containing a peptide and a physiologically acceptable carrier. These compositions typically contain one or more of the disclosed Mtb peptides (or a fragment thereof that binds MHC or a fusion protein thereof) in an amount ranging from about 1 µg to about 100 µg, such as from about 10 µg to about 50 µg in a volume of 0.1 ml. The carrier employed in a pharmaceutical composition can be a saline solution with appropriate preservatives, such as phenol and/or TWEEN80^{™}.

Generally, the polypeptide or peptide employed in a skin test is of sufficient size such that it remains at the site of injection for the duration of the reaction period. In some cases, a peptide that is at least nine amino acids in length is sufficient. Without being bound by theory, the polypeptide or peptide is broken down by macrophages within hours of injection to allow presentation to T-cells. Such polypeptides or peptides can contain repeats of one or more of the above disclosed sequences and/or other immunogenic or non-immunogenic sequences.

The method further includes detecting the presence of T cells that specifically recognize the amino acid sequence of the peptide in the subject, wherein the presence of T cells that specifically recognize the amino acid sequence of the at least one peptide at or adjacent the site of administration indicates that the subject has an immune response to *Mycobacterium tuberculosis.* The determination of the recognition of the at least one peptide by the T cells can be measured in vivo. In some cases, the at least one peptide is administered to the individual and then a response which indicates recognition of the at least one peptide may be measured. In one aspect the at least one peptide is administered intradermally, typically in a similar manner to the Mantoux test. The at least one peptide can be administered epidermally. The at least one peptide may be administered by needle, such as by injection, but can be administered by other methods such as ballistics, for example the ballistics techniques which have been used to deliver nucleic acids. Published EPC Application No. EP-A-0693119 describes techniques which can typically be used to administer the at least one peptide. In several examples, from 0.001 to 1000 µg, for example from 0.01 to 100 µg or 0.1 to 10 µg of at least one peptide is administered. Alternatively, an agent can be administered which is capable of providing the at least one peptide in vivo. Thus, a polynucleotide capable of expressing the at least one peptide can be administered. The at least one peptide is expressed from the polynucleotide in vivo and recognition of the at least one peptide in vivo may be measured. In some cases, from 0.001 to 1000 µg, for example from 0.01 to 100 µg or 0.1 to 10 µg of polynucleotide is administered.

The at least one peptide may comprise an immunogenic amino acid sequence. The at least one peptide may comprise an amino acid sequence derived from the amino acid sequence of a polypeptide produced by any *Mtb* strain. The amino acid sequence of the at least one peptide may correspond to any portion of the full-length polypeptide. In some cases, the at least one peptide is formed by truncating a full-length polypeptide. In some cases, the polypeptide is ctpF (Accession No: ANZ82682), PPE15 (Accession No: AOE35388), PPE68 (Accession No: AOE38353), PE3 (Accession No: AOE34479), irtA (Accession No: ANZ82019), Rv0219 (Accession No: CCP42947), PE12 (Accession No: AOE35536), esxJ (Accession No: CCE36570), esxG (Accession No: CCE35829), PE_PGRS50 (Accession No: CCP46166), or PPE51 (Accession No: AOE37558).

In certain aspects, the at least one peptide comprises an amino acid sequence set forth in: RPELPVRNYIRIPAL (SEQ ID NO:1); LHEARTAALNLFVVV (SEQ ID NO: 2); YQTYMYTGQYDGY (SEQ ID NO: 6); IAPHFVRVRMVSPTLF (SEQ ID NO: 7); RRRCRPWLHRR (SEQ ID NO: 11); YVGLYLPFLDMSF (SEQ ID NO: 14); GPRYGFKPTVM (SEQ ID NO: 4); NEIRALMAL (SEQ ID NO: 15); YYLGEPIVQAVL (SEQ ID NO: 8); WMGPASMAMVAAAQP (SEQ ID NO: 3); RQFHHPLIYVLLV (SEQ ID NO: 13); TQALQALTIPSF (SEQ ID NO: 12); TLFEKLEPMASIL (SEQ ID NO: 5); orAMSAQAFHQGESSAAF (SEQ ID NO: 10).

In certain aspects, the at least one peptide is a single peptide comprising the amino acid sequence set forth in SEQ ID NO:3, or is at least two peptides, at least three peptides, at least four peptides, at least five peptides, at least six peptides, at least seven peptides, at least eight peptides, at least nine peptides, at least ten peptides, at least eleven peptides, at least twelve peptides, at least thirteen peptides, or fourteen peptides, each (further) peptide independently comprising the amino acid sequence set forth in SEQ ID NO:1, SEQ ID NO:2, SEQ ID NO:6, SEQ ID NO:7, SEQ ID NO:11, SEQ ID NO: 14, SEQ ID NO: 4, SEQ ID NO: 15, SEQ ID NO: 8, SEQ ID NO: 3, SEQ ID NO: 13, SEQ ID NO: 12, SEQ ID NO: 5, or SEQ ID NO: 10.

The length of the at least one peptide may vary. The at least one peptide may range in length from 9 to 12 amino acids, from 9 to 18 amino acids, from 9 to 20 amino acids, or from 9 to 25 amino acids. In some cases, the at least one peptide may range in length from 15 amino acids to 886 amino acids, from 15 to 18 amino acids, from 11 to 18 amino acids, from 13 to 466 amino acids, from 16 to 315 amino acids, from 12 to 16 amino acids, from 11 to 33 amino acids, from 13 to 16 amino acids, from 13 to 21 amino acids, or from 9 to 16 amino acids. In some cases, the at least one peptide includes an additional 1 to 5 amino acids that extends the length of the at least one peptide at the N terminus, where the additional amino acids are derived from the corresponding amino acid sequence of the *Mycobacterium* polypeptide from which the at least one peptide is derived. In some cases, the at least one peptide includes an additional 1 to 5 amino acids that extends the length of the at least one peptide at the C terminus, where the additional amino acids are derived from the corresponding amino acid sequence of the *Mycobacterium* polypeptide from which the at least one peptide is derived.

Other aspects of the at least one peptide are disclosed in the preceding sections and are not repeated here for sake of brevity.

### Treatment

Also described is a method for treating an Mtb infection. In some cases, where the present methods indicate the subject has an immune response to *M. tuberculosis,* the subject may be treated for preventing tuberculosis and/or ameliorating one or more symptoms of tuberculosis, if present. The treatment may be administered to the patient before or after the present methods indicate the subject has an immune response to Mycobacterium tuberculosis.

Treatments for preventing tuberculosis and/or ameliorating one or more symptoms of tuberculosis include, but are not limited to, administering a therapeutically effective amount of an anti-tuberculosis agent to an individual. In some cases, the agent is administered to the subject for a therapeutically effective amount of time. In some cases, the agent is administered to a subject in a therapeutically effective dose regimen. In some cases, the treatment includes administering a therapeutically effective amount of an anti-microbial agent such as an antibiotic. In some cases, one or more anti-tuberculosis agents effective for treating tuberculosis are administered to the subject, which agents may include, e.g., isoniazid, rifampin, pyrazinamide, ethambutol, rifapentine, streptomycin, cycloserine, capreomycin, ρ-Aminosalicylic acid, ethionamide, bedaquiline, or a combination thereof. In some cases, a combination of anti-tuberculosis agents is administered to the subject at a therapeutically effective dose for a therapeutically effective period of time, where the combination may include, e.g., isoniazid plus rifampin; rifapentine plus isoniazid; isoniazid, rifampin, plus pyrazinamide; and/or isoniazid, rifampin, ethambutol, plus pyrazinamide. In some cases, an anti-tuberculosis therapy regimen includes an intensive phase of two months during which a combination of anti-tuberculosis agents at a therapeutically effective dose are administered to the subject, followed by a continuation phase of four or seven months where another combination of anti-tuberculosis agents at a therapeutically effective dose are administered to the subject.

Described is a method comprising: contacting a biological sample, as provided herein, comprising T cells from a subject, wherein the subject is suspected of having T cells that specifically recognize a *M*. *tuberculosis* antigen, with at least one 9 to 18 amino acids long peptide comprising an amino acid sequence having at least 70% identity to the amino acid sequence: RPELPVRNYIRIPAL (SEQ ID NO:1); LHEARTAALNLFVVV (SEQ ID NO: 2); YQTYMYTGQYDGY (SEQ ID NO: 6); IAPHFVRVRMVSPTLF (SEQ ID NO: 7); RRRCRPWLHRR (SEQ ID NO: 11); YVGLYLPFLDMSF (SEQ ID NO: 14); GPRYGFKPTVM (SEQ ID NO: 4); NEIRALMAL (SEQ ID NO: 15); YYLGEPIVQAVL (SEQ ID NO: 8); WMGPASMAMVAAAQP (SEQ ID NO: 3); RQFHHPLIYVLLV (SEQ ID NO: 13); TQALQALTIPSF (SEQ ID NO: 12); TLFEKLEPMASIL (SEQ ID NO: 5); or AMSAQAFHQGESSAAF (SEQ ID NO: 10); detecting the presence or absence of T cells in the biological sample that specifically recognize the amino acid sequence of the at least one peptide; and treating the subject by administering a therapeutically effective amount of an agent against *M. tuberculosis.* The described method may further comprise detecting the presence of T cells that specifically recognize a *M. tuberculosis* antigen after the administering, wherein the detecting is performed using the at least one peptide as disclosed herein; detecting the presence or absence of T cells that specifically recognize a *M. tuberculosis* antigen; and discontinuing the treatment when a significant level of T cells that specifically recognize a *M. tuberculosis* antigen are not detected or continuing the treatment when a significant level of T cells that specifically recognize a *M. tuberculosis* antigen is detected.

The subject may be a subject who is suspected of having T cells that specifically recognize a *M. tuberculosis* antigen. The antigen may be at least one peptide comprising an amino acid sequence having at least 70% identity to the amino acid sequence set forth in any one of SEQ ID NOs: 1-15. In some cases, the subject is suspected of having an immune response to *M. tuberculosis.* In some cases, the subject suspected of having T cells that specifically recognize a *M*. *tuberculosis* antigen has been exposed to *M. tuberculosis,* e.g., is a subject who has an *M. tuberculosis* infection and may have a latent tuberculosis or active tuberculosis. In some cases, the subject suspected of having T cells that specifically recognize a *M*. *tuberculosis* antigen is a healthcare professional who may have been exposed to *M. tuberculosis* in a clinical setting. In some cases, the subject suspected of having T cells that specifically recognize a*M*. *tuberculosis* antigen lives in or has traveled to a region with a high incidence of infections caused by *M. tuberculosis.* In some cases, the subject suspected of having T cells that specifically recognize a *M. tuberculosis* antigen presents symptoms of an *M. tuberculosis* infection. In some cases, the subject suspected of having T cells that specifically recognize a*M*. *tuberculosis* antigen lives with or is a caretaker for an individual who has an *M. tuberculosis* infection. In some cases, the subject suspected of having T cells that specifically recognize a*M*. *tuberculosis* antigen is a subject who had previously received a treatment for *M. tuberculosis* infection.

Described is a method for treating a subject having an immune response to *Mycobacterium tuberculosis,* the method comprising: contacting a biological sample, as provided herein, comprising T cells from the subject with at least one 8 or 9 to 18 amino acids long peptide comprising an amino acid sequence having at least 70% identity to the amino acid sequence: RPELPVRNYIRIPAL (SEQ ID NO:1); LHEARTAALNLFVVV (SEQ ID NO: 2); YQTYMYTGQYDGY (SEQ ID NO: 6); IAPHFVRVRMVSPTLF (SEQ ID NO: 7); RRRCRPWLHRR (SEQ ID NO: 11); YVGLYLPFLDMSF (SEQ ID NO: 14); GPRYGFKPTVM (SEQ ID NO: 4); NEIRALMAL (SEQ ID NO: 15); YYLGEPIVQAVL (SEQ ID NO: 8); WMGPASMAMVAAAQP (SEQ ID NO: 3); RQFHHPLIYVLLV (SEQ ID NO: 13); TQALQALTIPSF (SEQ ID NO: 12); TLFEKLEPMASIL (SEQ ID NO: 5); AMSAQAFHQGESSAAF (SEQ ID NO: 10); or QTVEDEARRMW (SEQ ID NO: 9); and detecting the presence of T cells that specifically recognize the amino acid sequence of the at least one peptide; and treating the subject having an immune response to *Mycobacterium tuberculosis* with anti-tuberculosis therapy.

In some cases, the contacting of a biological sample, as provided herein, includes performing a diagnostic assay, immunoassay, or detection assay, e.g., ELISA, as provided herein, where the biological sample is contacted with at least one of the peptides of the present disclosure (e.g., SEQ ID NOs: 1-15). The assay may produce a detectable signal, e.g., fluorescence or the presence of an enzymatic activity, as provided herein. In some cases, the presence of a detectable signal indicates that the biological sample contains T cells that specifically recognize the amino acid sequence of at least one peptide, at least two peptides, at least three peptides, at least four peptides, at least five peptides, at least six peptides, at least seven peptides, at least eight peptides, at least nine peptides, at least ten peptides, at least eleven peptides, at least twelve peptides, at least thirteen peptides, at least fourteen peptides, or fifteen peptides of the present disclosure. In some cases, the presence of a detectable signal produced from the contacting of the biological sample with at least one peptide, at least two peptides, at least three peptides, at least four peptides, at least five peptides, at least six peptides, at least seven peptides, at least eight peptides, at least nine peptides, at least ten peptides, at least eleven peptides, at least twelve peptides, at least thirteen peptides, at least fourteen peptides, or fifteen peptides of the present disclosure indicates that the subject has an immune response to *M*. *tuberculosis.* The at least one peptide are as disclosed herein and description of the at one peptide is not repeated in interest of brevity.

### KITS

Also provided by the present disclosure are immunoassay kits for detecting the presence of T cells in a biological sample of a subject or at an intradermal site of administration in the subject indicative of an immune response to *Mycobacterium tuberculosis* in the subject. The immunoassay kit includes at least one peptide one 9 to 18 amino acids long peptide comprising an amino acid sequence having at least 70% identity to the amino acid sequence set forth in SEQ ID NO: 3, as disclosed herein. In some cases, the at least one peptide is immobilized on a solid support.

The kits may include one or more reagents useful in practicing the methods of the present disclosure.

### UTILITY

The methods and kits of the present disclosure find use in any application which includes monitoring the progression of an Mtb infection and/or effectiveness of Mtb therapy.

In certain aspects, assays as described above for the detection of an immune response to *Mycobacterium tuberculosis* infection may be performed over time, and the change in the level of T cells evaluated. For example, the method can be performed about every 12, 24, 36, 48, 60, or 72 hours for a specified period, such as over months or weeks, and thereafter performed as needed. The *Mycobacterium* infection may be progressing in those patients in whom the level of T cells increases over time. In contrast, the *Mycobacterium* infection is not progressing when the level of T cells either remains constant or decreases with time. In this manner, the effectiveness of a particular therapeutic regimen can be assessed.

In another aspect, the presence of T cells, such as CD8⁺ T cells and/or CD4⁺ T cells that specifically recognize at least one Mtb peptide is assessed in a subject. The subject is administered a therapeutic protocol. The presence of the T cells that specifically recognize the at least one Mtb peptide is then assessed. A decrease or no change in the amount of CD8⁺ T cells and/or CD4⁺ T cells that specifically recognize the Mtb polypeptide as compared to the amount of the CD8⁺ T cells and/or CD4⁺ T cells, respectively, that specifically recognize the at least one Mtb polypeptide prior to the administration of the therapeutic protocol indicates that the therapeutic protocol is not effective. An increase in the amount of the CD8⁺ T cells and/or CD4⁺ T cells that specifically recognize the at least one Mtb polypeptide as compared to the amount of the CD8⁺T cells and/or CD4⁺ T cells that specifically recognize the at least one Mtb polypeptide prior to the administration of the therapeutic protocol indicates that the therapeutic protocol is effective.

It should be noted that for any of the above-described assays, to improve sensitivity, multiple *Mycobacterium* markers may be assayed within a given sample. It will be apparent that the assays disclosed herein can be used in combination. Thus, sets of *Mycobacterium* peptides, and combinations of assays can be for optimal sensitivity and specificity.

Numerous other assay protocols exist that are suitable for use with the polypeptides of the present disclosure.

### EXAMPLES

It is understood that the examples and embodiments described herein are for illustrative purposes only.

### EXAMPLE 1

### Methods and Materials

### Production of Library 1

The open source neural network platform NetMHC which has been shown to accurately predict MHC binding (1, 2), was used to predict the binding affinity of all possible 9-, 10-, and 11-mer peptides to the 12 major HLA supertypes (A1, A2, A3, A24, A26, B7, B8, B27, B39, B44, B58, and B62) which covers 99.6% of all ethnic groups. First, the amino acid (a.a.) sequences of 45 immunodominant CD8 antigens were evaluated for 9-, 10-, and 11-mer peptides that had a predicted binding affinity of ≤ 50 nM for at least one HLA supertype. Using this filter 3817 peptides were identified. Second, this analysis was repeated, where the analysis included evaluating for 9- 10-, and 11-mer peptides that had a predicted binding affinity of ≤ 50 nM for at least 3 HLA supertypes. Using this filter 1038 peptides were identified.

It has been found that the vast majority of immunodominant epitopes are restricted by HLA-B alleles (3). Therefore to ensure that the set of putative binding peptides was enriched for peptides binding to HLA-B alleles, the analysis was repeated, where the analysis included evaluating for 9- 10-, and 11-mer peptides that had a predicted binding affinity of ≤ 50 nM for at least 2 HLA supertypes, and that included at least 1 predicted to bind to an HLA-B supertype. Using this filter 615 peptides were identified. Finally, excluding duplicate peptides between the two sets of peptides, a total of 1392 peptides were identified. This approach was used to map clusters or regions of the CD8 antigens that contain multiple putative epitopes (9-, 10-, and 11-mers). A cluster was defined as at least three 9-, 10-, or 11-mer peptides separated by no more than 1 a.a. The density of the cluster was defined as the number of 9-, 10- or 11-mer peptides within cluster. Regarding the entire analysis, of the 1392 peptides with predicted binding affinity ≤ 50 nM, 639 of these 9-, 10-, or 11-mer peptides were mapped to a total of 138 clusters, which ranged in length from 11 to 27 a.a. Seventy eight percent (108/138) of the clusters contained at least one peptide predicted to bind to HLA-B. The average density of the clusters was 4.6 putative epitopes per cluster, ranging from 3 to 21.

It has been found that many immunodominant CD8 epitopes demonstrate very high affinity binding (≤ 10 nm) to the restricting HLA allele (3). Therefore, in order not to miss these immunodominant epitopes, all putative 9-, 10-, or 11-mers predicted to have very high binding affinity (≤ 10 nm) were included, regardless of whether or not they were contained in a cluster. Specifically, the NetMHC analysis of all 45 amino acid sequences was repeated for 9-, 10-, or 11-mers that had a predicted binding affinity of ≤ 10 nm for at least 1 HLA supertype. Using this filter 27 peptides were identified. For the entire analysis, 16 of these 27 peptides mapped within a cluster, leaving 11 peptides with very high binding affinity not mapped to a cluster. The next step included creating a peptide library of 15-mers overlapping by 11 a.a. that represent all 138 clusters (155 peptides) plus the individual 11 peptides (six 9-mers, one 10-mer, and four 11-mers) with very high binding affinity not mapped to a cluster. As 155 overlapping 15-mers were required to represent the clusters, the final peptide library included 166 peptides. This peptide library contained a total of 650 putative epitopes with high (n= 623, ≤ 50 nM) and very high (*n =* 27, ≤ 10 nm) predicted binding affinity.

### Individual Validation of peptides in Library 1 in Humans Infected with Mtb

While prediction tools for peptide/MHC binding have greatly improved, many peptides that bind HLA with high affinity are not necessarily immunogenic (3). Therefore, predicted peptide epitopes must still be validated for recognition by CD8+ T cells ex vivo. The human T cell response is the best guide to antigen discovery as some immunodominant epitopes detected using Mtb-specific CD8+ T cells would not have been predicted by peptide binding prediction tools. In addition, using this approach the following was ensured: (1) an unbiased assessment of the prediction tools; (2) insight into the recognition across ethnically diverse subjects; and (3) assurance that the CD8+ T cell response is specific for Mtb infection. Therefore, the recognition of individual peptides of the peptide library (*n* = 166) selected as described above by CD8+ T cells from a racially diverse cohort of adults with LTBI and uninfected adults was studied.

Healthy, non-pregnant adults, ages 18-65 yrs were recruited for study. Individuals with LTBI must have had a history of a positive *tuberculin* skin test (TST) or positive Mtb interferon gamma release assay (IGRA) result. Uninfected individuals had a history of a negative TST and no prior exposure to TB. A total of 21 subjects were enrolled into the LTBI and uninfected groups. The cohorts were racially diverse and included Asian, African American and Caucasian racial groups. Each consented individual had one unit of blood drawn at a single visit. Blood (20 ml) was then used for HIV serology and an IGRA, the Quantiferon in-tube^{®} assay (QFT-IT), to test for LTBI. Individuals who were positive for HIV were excluded from the study. Regarding definition of LTBI, subjects with a positive QFT-IT regardless of history of TST result were assigned to the LTBI cohort. Subjects who were QFT-IT negative and have a history of negative TST were classified as uninfected. To avoid misclassification bias, subjects with a history of a positive TST and a negative QFT-IT were excluded.

LTBI subjects had positive assays for a large proportion of the individual peptides in peptide library. Therefore, a more robust definition of immunodominance, which took into account not only how-commonly-recognized, but also how strongly an epitope is recognized, as reflected by the magnitude of the response, was used.

First, for each individual in the LTBI cohort, the response to each individual peptide was ranked by the number of SFU and a strongly recognized peptide was defined as one eliciting a response ranked in the top 5% of the individual's positive responses. For example, if an individual had a positive assay for 40 of the 166 peptides, then the 2 peptides eliciting the most SFU would be in the top 5% of this individual's responses and denoted as strongly recognized. Second, the frequency a particular peptide elicited a positive assay in the LTBI cohort was evaluated. A commonly recognized peptide is defined as one that elicited positive assays in at least 3 individuals with LTBI.

### Generation of Library 2

To develop another library of Mtb peptides to evaluate for their potential to elicit an immune response, the entire peptide sequences of 18 known immunodominant antigens were used to create an optimal list of clustered peptides predicted to bind with high affinity to 12 HLA supertypes. Using a 100 nm cut off for binding affinity, 1111 peptides consisting of 9-, 10-, or 11mers that bound to at least one of the 12 HLA supertypes were found. Next, to identify clusters of strong binders, 'hotspots' defined as at least three 9, 10, or 11mer peptides separated by 1 a.a. each were identified. The result was 134 clusters. The density of the cluster is defined as the number of peptides (9, 10, and 11mer predicted binders) contained within the cluster. The density ranged from 3 to 19 peptides. Seventy five (55%) of the clusters had a modest density of 3 peptides. From these clusters, 15mers with an 11 amino acid overlap were constructed; this resulted in 154 peptides as a starting point for the 2^{nd} peptide library.

To ensure that the list included extremely high affinity binders (here, ≤ 20 nm) the list of 18 antigens was re-analyzed but the binding affinity was set at < 20nm. The < 20nm binding list was compared with the analysis of the < 100nm binding list. Fifty-three percent of the peptides meeting the < 20nm threshold were previously included in the < 100nm list. The goal was to reach a peptide library with ~170 peptides by prioritizing all high affinity binders (< 20nm) peptides and peptides with a higher density. Existing clusters were extended to include 20 additional peptides with a high binding affinity (< 20nm) in close proximity to a cluster. For those clusters with a low density (n =3), clusters that were characterized by 9,10 or 11mers starting at subsequent positions (e.g. for antigen A, a 9mer at position 15, a 10mer at position 16 and a 11mer at position 17 were prioritized higher than a 9, 10 and 11mer all starting at position 15) were prioritized. This allowed the inclusion of all binders (< 20nm) that were not already included in a cluster from the < 100nm analysis. Using this approach, a 2nd peptide library with 176 peptides, 80% of which included at least one high affinity (< 20nm) binder, was developed.

As with Library 1, the predicted peptide clusters were screened for recognition by CD8⁺ T cells from adults with (*n* = 21) and without (*n* = 21) LTBI using the CD8/others ELISPOT assay, which allowed measurement of CD8+ T cell responses without the need for additional antigen presenting cells.

### Isolating PBMC and CD8+ T cells

Blood from consented donors was drawn into multiple 8.5 mL whole blood acid citrate dextrose tubes and processed immediately after completing the draw. The blood was transferred to 50 mL conical tubes (Falcon^{®}, Corning) and diluted 1: 1 with sterile phosphate buffered saline (PBS), pH 7.4 (Invitrogen). The mixture was underlayed with Histopaque 1077 (Sigma) and the tubes centrifuged for 25 minutes at 2500 at room temperature (RT) in an Allegra 6R temperature controlled centrifuge (Beckman). After centrifugation, the buffy coat containing the peripheral blood mononuclear cells (PBMC) was collected from each tube was pooled in clean 50 mL conical tubes. The cells were then washed with a PBS, 1% fetal bovine serum (FBS; Hyclone, ThermoScientific) solution and centrifuged for 10 minutes at 1800 rpm at RT. The supernatant was aspirated and discarded. The cell pellet was again resuspended in PBS, 1% FBS and centrifuged for 10 minutes at 1300rpm at RT. The supernatant was aspirated off of the pellet and discarded and the previous wash step was repeated. The supernatant was aspirated off and discarded. The resulting pellet was resuspended in 5 ml RPMI, 10% FBS and counted. The cells were then centrifuged at 1300 rpm for 10 min at RT. The cell pellet was resuspended with freezing media (50% FBS, 40% RPMI, 10% DMSO) and aliquoted into cryovials. The vials were placed into freezing containers (Mr. Frosty^{®}, ThermoScientific) and placed in a lab freezer at -80C and transferred the next day to a liquid nitrogen storage system.

### Preparation of Coated ELISPOT Plates

To prepare 96-well, enzyme-linked immunospot (ELISPOT) plates, anti-IFNγ antibody (Mabtech) was diluted with ELISPOT 1 Buffer (sodium carbonate/bicarbonate, pH 9.6) 1: 1000. The plates were allowed to incubate overnight in the refrigerator after 50 µL of the diluted-antibody solution was added to each well. The following day, the coating solution was discarded and each plate was washed by filling each well with 200 µL of sterile PBS and allowing the plate to incubate for 10 min at room temperature. The solution was discarded and the wash step was repeated two times. The surface of each well was blocked using 100µL of a blocking solution 10% HuS Media (10% human serum in RPMI supplemented with L-glutamine and gentamicin) and incubated for an hour at room temperature.

### Preparation of PBMC for CD8⁺ T cell Selection

For each donor's cells being processed, DNase was added to two 50 mL conical tubes containing 45 mL of 10% FBS Media (10% Fetal Bovine Serum in RPMI supplemented with L-glutamine and gentamicin). Donor's PBMC were thawed in a 37C waterbath. The thawed cells were transferred into the two conical tubes evenly and centrifuged for 10 min at 1300 rpm. The supernatant was aspirated off the pellets which were then resuspended and mixed thoroughly in a total of 50 mL 10% HuS Media with 1.5 mL 5mg/ml DNase added and then combined into one conical tube. The cells were counted and then centrifuged at 1300 rpm for 10 min. The pellet was resuspended at 1x10⁸ cells/mL in cold 2% FBS in PBS, 1mM EDTA with 300µL/10mL DNase.

### Positive Selection of CD8⁺ T cells

Resuspended PBMC were transferred to 5 mL tubes and EasySEP^{™} Positive Selection Cocktail (Stemcell Technologies, Inc.) was added at 100 µL/mL of cells and mixed well. After incubating at room temperature for 15 minutes, magnetic nanoparticles were added to the tube at 100 µL/mL of cell suspension and the mixture was incubated at room temperature for 10 min. The cell suspension was brought up to volume with 2% FBS in PBS, 1mM EDTA and mixed gently by pipetting. The tube was placed into a magnet (EasySEP^{™}), and allowed to incubate. After 5 minutes, the magnet was inverted to pour off the supernatant fraction. After removing the tubes from the magnet, 2% FBS in PBS, 1 mM EDTA was added and mixed gently by pipetting. The tube was placed back in the magnet twice more and the previous steps washing steps repeated. The cells were then resuspended in cell medium and centrifuged for 10 min at 1300 rpm. The cell pellet was resuspended in 10% human serum in RPMI and mixed for counting.

### Preparing Dendritic Cells

The media in the cell culture flasks containing dendritic cells (DC) was pipetted off the cell layer and put into a 50 mL conical tube. Ten milliliters of PBS were added to the flask which was then rocked gently. The PBS was pulled off and added to the media in the 50 mL tube. Ten milliliters of cell-dissociation solution was added to the flask and the flask was incubated at 37C for 15 minutes. The flask was then whacked firmly and the solution was pipetted off and added to the 50 mL conical which was then spun at 1800 rpm for 15 min. After the spin, the media was aspirated off of the pellet which was then resuspended in 5 mL of 10% HuS media for counting. The dendritic cells were resuspended at 4x10⁵ cells/mL. A 2 ng/mL solution of Interleukin 2 was added to the suspension for a final concentration of 0.5 ng/mL.

### ELISPOT Assay

Excluding "CD8-only" wells, 50 µL of the prepared dendritic cells were added to experimental wells and 100 µL of media was added. Following addition of the DC, 100 µL of CD8⁺ T cells were added to appropriate wells ("DC only" cells were excluded). Twenty-five microliters of each test peptide was added to separate experimental wells such that each experimental well contained only one peptide sequence. Phytohemagglutinin (PHA) was added to positive control wells. Negative control wells, termed "media" or "media-only" wells contained CD8⁺ T cells and DC, however only cell culture medium was added to the wells - peptides or mitogens were not added to these wells. The plate was allowed to incubate over night at 37C in a CO₂ incubator. To begin development of the plates, the cells and media were discarded by flicking the plates into a biohazard bag and the plates were washed six times with PBS-0.05% Tween. Interferon gamma antibody with conjugated horseradish peroxidase was diluted to 1 µg/mL in coating buffer (PBS, Bovine Serum Albumin, Tween 20) and 100 µL/well of the mix was added to each well and incubated at room temperature for two hours. After incubation, the plates were washed with PBS/Tween six times and then incubated with PBS for 15 minutes. Another round of six PBS Tween washes were performed. Following three rounds of washes with PBS, excess PBS was removed from the plates by smacking the plates on towels. The plates were then developed using 100 µL/well of HRP substrate. The plates were allowed to develop at room temperature and upon completion were washed with water for between 4 and 10 minutes and then air dried. The developed plates were analyzed with an ELISPOT reader (AID GmbH). ELISPOT data is reported to ELISPOT reader users as spot forming units (SFU). Each SFU represents a single, activated cell secreting interferon gamma.

As described above, peptides derived from immunodominant Mtb proteins were synthesized to include multiple predicted, high-affinity T cell epitopes contained within the amino acid sequence of the peptide. Each peptide synthesized was assayed using interferon gamma ELISPOT assays performed in 96-well plates using CD8+ T cells and dendritic cells as antigen presenting cells. After the plates were developed and read, data from each plate was exported from the ELISPOT reader in a Microsoft Excel spreadsheet.

Spot forming unit data from each plate's media-only wells were averaged and the result was subtracted from each experimental and control well SFU count to provide a mediasubtracted SFU count for each well. Responses in the experimental wells were considered positive if the SFU count for the well was (1) equal to, or greater than, a value that was two times the standard deviation from the media-only well average and (2) ten or more SFU after the media-only average was subtracted from the raw count. For each peptide tested, a "Sensitivity" and "Specificity" score for each peptide was calculated. Sensitivity scores were determined by calculating the proportion of donor samples that demonstrated a positive response to the same peptide sequence when compared to the total number of donors. Sensitivity scores of "0" indicate peptides that did not elicit a positive CD8⁺ T cell response using the criteria explained previously. The Specificity score was calculated by determining the proportion of positive wells for each peptide across all uninfected donors and subtracting that proportion from one (1.0). Therefore, a Specificity score of "1" means that zero uninfected donors responded to the peptide pool. Conversely, a Specifity score of "0" would mean that all of the uninfected donors responded to the relevant peptide. Peptides that demonstrate antigenicity in T cells from donors with a history of Mtb infection while maintaining a high specificity score are favorable for inclusion in T cell-based, diagnostic assays.

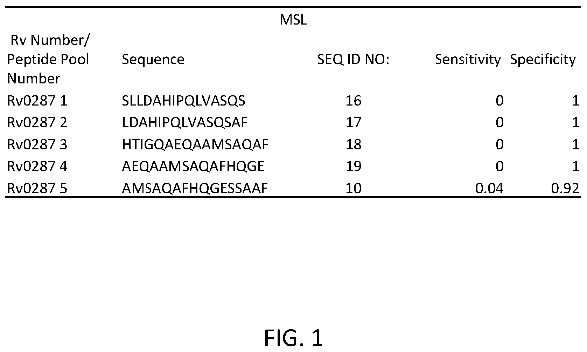

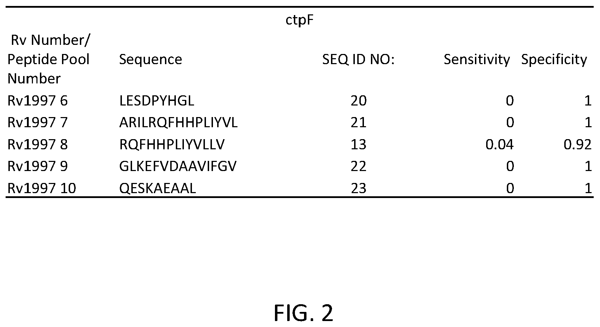

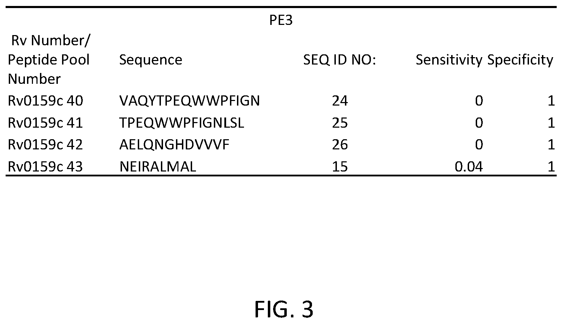

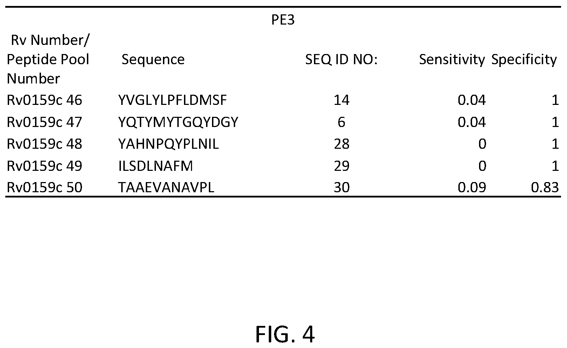

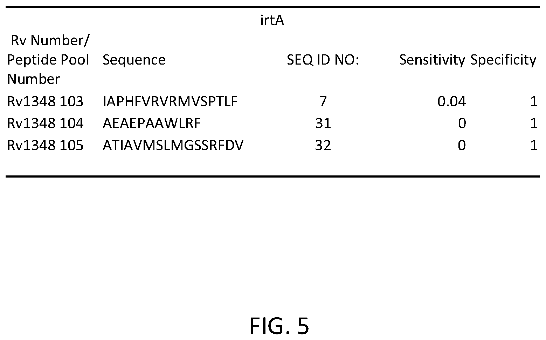

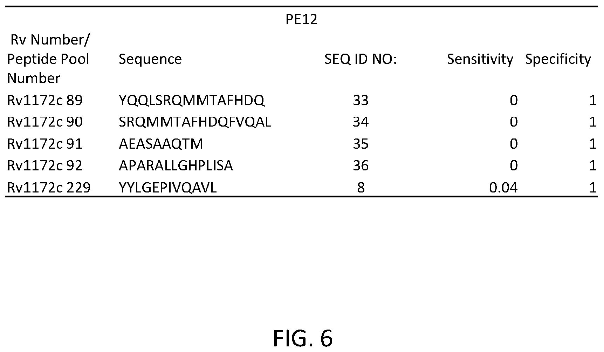

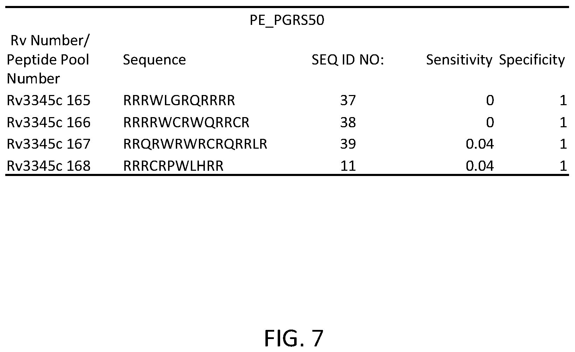

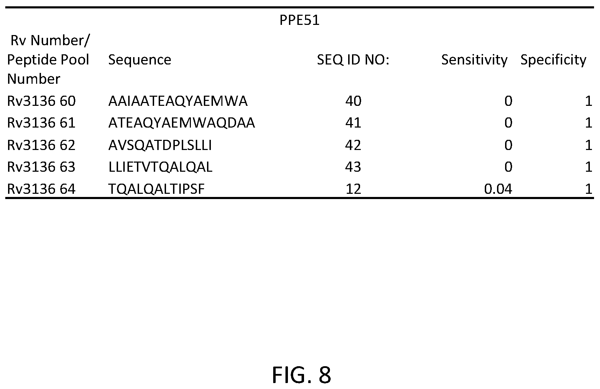

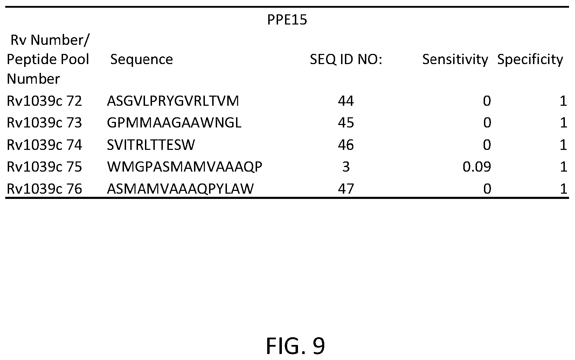

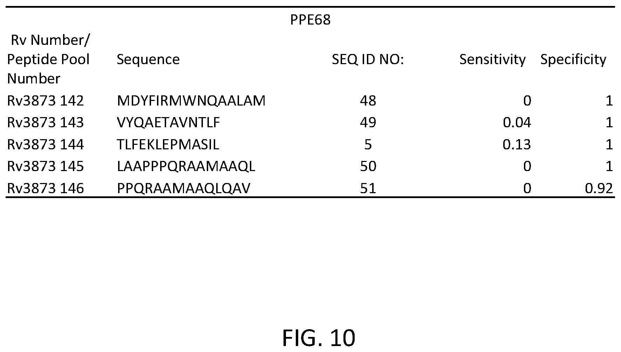

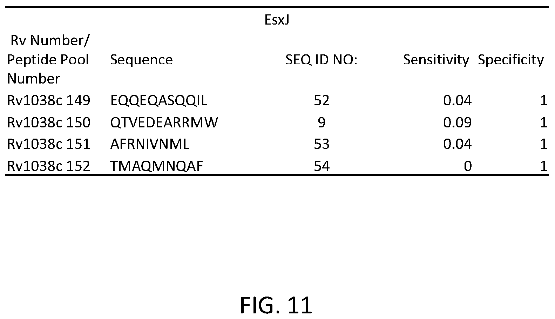

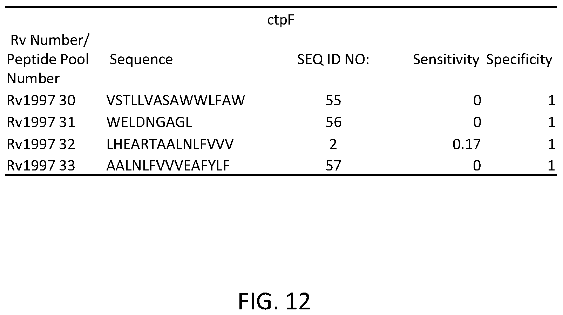

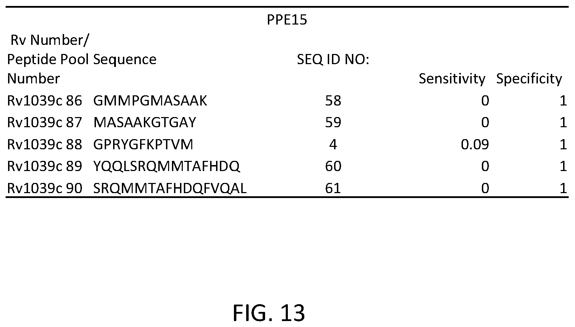

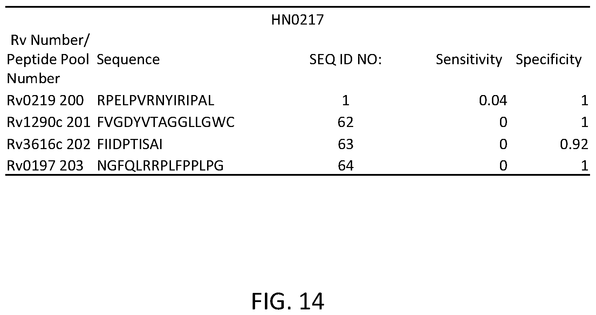

**FIGS. 1-14** depict the sensitivity and specificity for detection of Mtb exposure by peptides derived from Mtb antigens.

The donors in the latent tuberculosis infection LTBI (Mtb infected) had a positive IGRA, but did not have active disease (tuberculosis).

### References

1. Lund, O., M. Nielsen, C. Kesmir, A. G. Petersen, C. Lundegaard, P. Worning, C. Sylvester-Hvid, K. Lamberth, G. Roder, S. Justesen, S. Buus, and S. Brunak. 2004. Definition of supertypes for HLA molecules using clustering of specificity matrices. Immunogenetics 55:797-810.
2. Sette, A., and J. Sidney. 1999. Nine major HLA class I supertypes account for the vast preponderance of HLA-A and -B polymorphism. Immunogenetics 50:201-212.
3. Lewinsohn, D. A., E. Winata, G. M. Swarbrick, K. E. Tanner, M. S. Cook, M. D. Null, M. E. Cansler, A. Sette, J. Sidney, and D. M. Lewinsohn. 2007. Immunodominant tuberculosis CD8 antigens preferentially restricted by HLA-B. PLoS Pathog 3:1240-1249.

## Claims

1. A method for detecting an immune response to *Mycobacterium tuberculosis* in a subject, the method comprising:
contacting a biological sample comprising T cells from a subject suspected of having an immune response to *Mycobacterium tuberculosis* with at least one 8 to 18 amino acids long peptide comprising an amino acid sequence having at least 70% identity to the amino acid sequence:
WMGPASMAMVAAAQP (SEQ ID NO: 3); and
detecting the presence or absence of T cells in the biological sample that specifically recognize the amino acid sequence of the at least one peptide.

2. The method of claim 1, wherein the at least one peptide is a single peptide comprising an amino acid sequence having at least 70% identity to the amino acid sequence set forth in SEQ ID NO: 3 or wherein the at least one peptide is a single peptide consisting of the amino acid sequence set forth in SEQ ID NO: 3.

3. The method of claim 1, wherein the method comprises contacting the biological sample with at least two peptides, at least three peptides, at least four peptides, at least five peptides, at least six peptides, at least seven peptides, at least eight peptides, at least nine peptides, at least ten peptides, at least eleven peptides, at least twelve peptides, at least thirteen peptides, or fourteen peptides, wherein at least one of said peptides is 8 to 18 amino acids long and comprises an amino acid sequence having at least 70% identity to the amino acid sequence: WMGPASMAMVAAAQP (SEQ ID NO: 3) or consists of the amino acid sequence set forth in SEQ ID NO: 3, and wherein the other peptide or peptides is/are 8 to 18 amino acids long and each independently comprise an amino acid sequence having at least 70% identity to the amino acid sequence set forth in SEQ ID NO:1, SEQ ID NO:2, SEQ ID NO:6, SEQ ID NO:7, SEQ ID NO:11, SEQ ID NO: 14, SEQ ID NO: 4, SEQ ID NO: 15, SEQ ID NO: 8, SEQ ID NO: 13, SEQ ID NO: 12, SEQ ID NO: 5, SEQ ID NO: 10 or QTVEDEARRMW (SEQ ID NO:9), or consist of the amino acid sequence set forth in SEQ ID NO:1, SEQ ID NO:2, SEQ ID NO:6, SEQ ID NO:7, SEQ ID NO:11, SEQ ID NO: 14, SEQ ID NO: 4, SEQ ID NO: 15, SEQ ID NO: 8, SEQ ID NO: 13, SEQ ID NO: 12, SEQ ID NO: 5 or SEQ ID NO: 10.

4. The method of any of claims 1-3, wherein the biological sample from the subject is blood, isolated peripheral blood mononuclear cells, or isolated mononuclear cells.

5. The method of any of claims 1-4, wherein the biological sample comprises antigen presenting cells.

6. The method of any of claims 1-5, wherein the T cells are CD8+ T cells.

7. The method of claim 6, wherein the detecting comprises measuring secretion of a cytokine from the CD8+ T cells and/or measuring an amount of a cytokine inside of the CD8+ T cells.

8. The method of claim 7, wherein the cytokine is interferon-γ, TNF, and/or IP-10.

9. The method of any of claims 6-8, wherein the detecting comprises contacting the CD8+ T cells with Tetrameric MHC Class I/peptide complexes that specifically associate with a CD8+ T cell surface protein.

10. The method of any of claims 1-9, wherein the detecting comprises conducting an enzyme-linked immunospot assay or an enzyme-linked immunosorbent assay, optionally wherein the assay detects interferon-γ or IP-10.

11. At least one 9 to 18 amino acids long peptide for use in a method of diagnosing an immune response to *Mycobacterium tuberculosis* in vivo, the method comprising:
administering into the skin of a subject suspected of having an immune response to *Mycobacterium tuberculosis* an effective amount of the at least one 9 to 18 amino acids long peptide, comprising an amino acid sequence having at least 70% identity to the amino acid sequence:
WMGPASMAMVAAAQP (SEQ ID NO: 3); and
detecting presence of T cells that specifically recognize the amino acid sequence of the peptide in the subject.

12. The at least one 9 to 18 amino acids long peptide for use of claim 11, wherein the method comprises administering into the skin of the subject an effective amount of a single peptide, or at least two peptides, at least three peptides, at least four peptides, at least five peptides, at least six peptides, at least seven peptides, at least eight peptides, at least nine peptides, at least ten peptides, at least eleven peptides, at least twelve peptides, at least thirteen peptides, or fourteen peptides, wherein at least one of said peptides is 9 to 18 amino acids long and comprises an amino acid sequence having at least 70% identity to the amino acid sequence: WMGPASMAMVAAAQP (SEQ ID NO: 3) or consists of the amino acid sequence set forth in SEQ ID NO: 3, and wherein the other peptide or peptides is/are 9 to 18 amino acids long and each independently comprise an amino acid sequence having at least 70% identity to the amino acid sequence set forth in SEQ ID NO:1, SEQ ID NO:2, SEQ ID NO:6, SEQ ID NO:7, SEQ ID NO:11, SEQ ID NO: 14, SEQ ID NO: 4, SEQ ID NO: 15, SEQ ID NO: 8, SEQ ID NO: 13, SEQ ID NO: 12, SEQ ID NO: 5 or SEQ ID NO: 10, or consist of the amino acid sequence set forth in SEQ ID NO:1, SEQ ID NO:2, SEQ ID NO:6, SEQ ID NO:7, SEQ ID NO:11, SEQ ID NO: 14, SEQ ID NO: 4, SEQ ID NO: 15, SEQ ID NO: 8, SEQ ID NO: 13, SEQ ID NO: 12, SEQ ID NO: 5 or SEQ ID NO: 10, optionally wherein the T cells are CD8+ T cells.

13. The at least one 9 to 18 amino acids long peptide for use of claim 11 or 12, wherein the detecting the presence or absence of T cells comprises detecting a delayed type hypersensitivity reaction, optionally wherein the delayed type hypersensitivity reaction is measured by measuring redness, swelling, or induration of the skin at or adjacent the site of administration.

14. An immunoassay kit for detecting the presence of T cells in a biological sample of a subject or at an intradermal site of administration in the subject indicative of an immune response to *Mycobacterium tuberculosis* in the subject, the kit comprising at least one 9 to 18 amino acids long peptide comprising an amino acid sequence having at least 70% identity to the amino acid sequence:
WMGPASMAMVAAAQP (SEQ ID NO: 3).

15. The immunoassay kit of claim 14, wherein the kit comprises at least a single peptide, at least two peptides, at least three peptides, at least four peptides, at least five peptides, at least six peptides, at least seven peptides, at least eight peptides, at least nine peptides, at least ten peptides, at least eleven peptides, at least twelve peptides, or at least thirteen peptides, at least fourteen peptides, wherein at least one of said peptides is 9 to 18 amino acids long and comprises an amino acid sequence having at least 70% identity to the amino acid sequence: WMGPASMAMVAAAQP (SEQ ID NO: 3) or consists of the amino acid sequence set forth in SEQ ID NO: 3, and wherein the other peptide or peptides is/are 9 to 18 amino acids long and each independently comprise an amino acid sequence having at least 70% identity to the amino acid sequence set forth in SEQ ID NO:1, SEQ ID NO:2, SEQ ID NO:6, SEQ ID NO:7, SEQ ID NO:11, SEQ ID NO: 14, SEQ ID NO: 4, SEQ ID NO: 15, SEQ ID NO: 8, SEQ ID NO: 13, SEQ ID NO: 12, SEQ ID NO: 5 or SEQ ID NO: 10 or consist of the amino acid sequence set forth in SEQ ID NO:1, SEQ ID NO:2, SEQ ID NO:6, SEQ ID NO:7, SEQ ID NO:11, SEQ ID NO: 14, SEQ ID NO: 4, SEQ ID NO: 15, SEQ ID NO: 8, SEQ ID NO: 13, SEQ ID NO: 12, SEQ ID NO: 5 or SEQ ID NO: 10, optionally wherein the peptide is immobilized on a solid support.

## Patentansprüche

1. Verfahren zum Nachweis einer Immunantwort auf *Mycobacterium tuberculosis* in einem Subjekt, wobei das Verfahren umfasst:
Inkontaktbringen einer biologischen Probe, die T-Zellen von einem Subjekt umfasst, das im Verdacht steht, eine Immunantwort auf *Mycobacterium tuberculosis* zu haben, mit mindestens einem 8 bis 18 Aminosäuren langen Peptid, das eine Aminosäuresequenz umfasst, mit mindestens 70 % Identität mit der Aminosäuresequenz:
WMGPASMAMVAAAQP (SEQ ID NO: 3); und
Nachweisen des Vorhandenseins oder der Abwesenheit von T-Zellen in der biologischen Probe, die spezifisch die Aminosäuresequenz des mindestens einen Peptids erkennen.

2. Verfahren nach Anspruch 1, wobei das mindestens eine Peptid ein einzelnes Peptid ist, das eine Aminosäuresequenz mit mindestens 70 % Identität mit der in SEQ ID NO: 3 dargestellten Aminosäuresequenz umfasst, oder wobei das mindestens eine Peptid ein einzelnes Peptid ist, das aus der in SEQ ID NO: 3 dargestellten Aminosäuresequenz besteht.

3. Verfahren nach Anspruch 1, wobei das Verfahren das Inkontaktbringen der biologischen Probe mit mindestens zwei Peptiden, mindestens drei Peptiden, mindestens vier Peptiden, mindestens fünf Peptiden, mindestens sechs Peptiden, mindestens sieben Peptiden, mindestens acht Peptiden, mindestens neun Peptiden, mindestens zehn Peptiden, mindestens elf Peptiden, mindestens zwölf Peptiden, mindestens dreizehn Peptiden oder vierzehn Peptiden umfasst, wobei mindestens eines der Peptide 8 bis 18 Aminosäuren lang ist und eine Aminosäuresequenz umfasst, die mindestens 70% Identität mit der Aminosäuresequenz WMGPASMAMVAAAQP (SEQ ID NO: 3) aufweist oder aus der in SEQ ID NO: 3 dargestellten Aminosäuresequenz besteht, und wobei das andere Peptid oder die anderen Peptide 8 bis 18 Aminosäuren lang ist/sind und jeweils unabhängig voneinander eine Aminosäuresequenz umfassen, die mindestens 70 % Identität mit der Aminosäuresequenz aufweist, die in SEQ ID NO:1, SEQ ID NO:2, SEQ ID NO:6, SEQ ID NO:7, SEQ ID NO:11, SEQ ID NO: 14, SEQ ID NO: 4, SEQ ID NO: 15, SEQ ID NO: 8, SEQ ID NO: 13, SEQ ID NO: 12, SEQ ID NO: 5, SEQ ID NO: 10, oder QTVEDEARRMW (SEQ ID NO:9) dargestellt ist, oder aus der Aminosäuresequenz bestehen, die in SEQ ID NO:1, SEQ ID NO: 2, SEQ ID Nr. 6, SEQ ID Nr. 7, SEQ ID Nr. 11, SEQ ID Nr. 14, SEQ ID Nr. 4, SEQ ID Nr. 15, SEQ ID Nr. 8, SEQ ID Nr. 13, SEQ ID Nr. 12, SEQ ID Nr. 5 oder SEQ ID Nr. 10 dargestellt ist.

4. Verfahren nach einem der Ansprüche 1-3, wobei es sich bei der biologischen Probe aus dem Subjekt um Blut, isolierte mononukleäre Zellen aus peripherem Blut oder isolierte mononukleäre Zellen handelt.

5. Verfahren nach einem der Ansprüche 1-4, wobei die biologische Probe Antigen-präsentierende Zellen umfasst.

6. Verfahren nach einem der Ansprüche 1-5, wobei die T-Zellen CD8+ T-Zellen sind.

7. Verfahren nach Anspruch 6, wobei das Nachweisen das Messen der Sekretion eines Zytokins aus den CD8+ T-Zellen und/oder das Messen einer Menge eines Zytokins innerhalb der CD8+ T-Zellen umfasst.

8. Verfahren nach Anspruch 7, wobei das Zytokin Interferon-γ, TNF und/oder IP-10 ist.

9. Verfahren nach einem der Ansprüche 6-8, wobei das Nachweisen das Inkontaktbringen der CD8+ T-Zellen mit MHC Klasse I Tetramer/Peptid-Komplexen umfasst, die spezifisch mit einem CD8+ T-Zell-Oberflächenprotein assoziieren.

10. Verfahren nach einem der Ansprüche 1-9, wobei das Nachweisen die Durchführung eines Enzym-Linked-Immunospot-Assays oder eines Enzym-Linked-Immunosorbent-Assays umfasst, optional wobei der Assay Interferon-γ oder IP-10 nachweist.

11. Mindestens ein 9 bis 18 Aminosäuren langes Peptid zur Verwendung in einem Verfahren zur Diagnose einer Immunantwort auf *Mycobacterium tuberculosis* in vivo, wobei das Verfahren umfasst:
Verabreichen einer wirksamen Menge des mindestens einen 9 bis 18 Aminosäuren langen Peptids, das eine Aminosäuresequenz mit mindestens 70 % Identität mit der Aminosäuresequenz: WMGPASMAMVAAAQP (SEQ ID NO: 3) umfasst, in die Haut eines Subjekts, das im Verdacht steht, eine Immunantwort auf *Mycobacterium tuberculosis* zu haben; und
Nachweisen des Vorhandenseins von T-Zellen, die spezifisch die Aminosäuresequenz des Peptids in dem Subjekt erkennen.

12. Das mindestens eine 9 bis 18 Aminosäuren lange Peptid zur Verwendung nach Anspruch 11, wobei das Verfahren das Verabreichen einer wirksamen Menge eines einzelnen Peptids oder von mindestens zwei Peptiden, mindestens drei Peptiden, mindestens vier Peptiden, mindestens fünf Peptiden, mindestens sechs Peptiden, mindestens sieben Peptiden, mindestens acht Peptiden, mindestens neun Peptiden, mindestens zehn Peptiden, mindestens elf Peptiden, mindestens zwölf Peptiden, mindestens dreizehn Peptiden oder vierzehn Peptiden in die Haut des Subjekts umfasst, wobei mindestens eines der Peptide 9 bis 18 Aminosäuren lang ist und eine Aminosäuresequenz umfasst, die mindestens 70 % Identität mit der Aminosäuresequenz WMGPASMAMVAAAQP (SEQ ID NO: 3) aufweist, oder aus der in SEQ ID NO: 3 dargestellten Aminosäuresequenz besteht, und wobei das andere Peptid oder die anderen Peptide 9 bis 18 Aminosäuren lang ist/sind und jeweils unabhängig voneinander eine Aminosäuresequenz umfassen, die mindestens 70 % Identität mit der in SEQ ID NO: 1, SEQ ID NO: 2, SEQ ID Nr. 6, SEQ ID Nr. 7, SEQ ID Nr. 11, SEQ ID Nr. 14, SEQ ID Nr. 4, SEQ ID Nr. 15, SEQ ID Nr. 8, SEQ ID Nr. 13, SEQ ID Nr. 12, SEQ ID Nr. 5 oder SEQ ID Nr. 10 dargestellten Aminosäuresequenz aufweist oder aus der in SEQ ID NO: 1, SEQ ID NO: 2, SEQ ID Nr. 6, SEQ ID Nr. 7, SEQ ID Nr. 11, SEQ ID Nr. 14, SEQ ID Nr. 4, SEQ ID Nr. 15, SEQ ID Nr. 8, SEQ ID Nr. 13, SEQ ID Nr. 12, SEQ ID Nr. 5 oder SEQ ID Nr. 10 dargestellten Aminosäuresequenz bestehen, optional wobei es sich bei den T-Zellen um CD8+ T-Zellen handelt.

13. Das mindestens eine 9 bis 18 Aminosäuren lange Peptid zur Verwendung nach Anspruch 11 oder 12, wobei das Nachweisen des Vorhandenseins oder der Abwesenheit von T-Zellen das Nachweisen einer Überempfindlichkeitsreaktion vom verzögerten Typ umfasst, optional wobei die Überempfindlichkeitsreaktion vom verzögerten Typ durch Messen von Rötung, Schwellung oder Verhärtung der Haut an oder in der Nähe der Verabreichungsstelle gemessen wird.

14. Immunoassay-Kit zum Nachweis des Vorhandenseins von T-Zellen in einer biologischen Probe eines Subjekts oder an einer intradermalen Verabreichungsstelle in dem Subjekt, die eine Immunantwort auf *Mycobacterium tuberculosis* in dem Subjekt anzeigt, wobei das Kit mindestens ein 9 bis 18 Aminosäuren langes Peptid umfasst, das eine Aminosäuresequenz umfasst, mit mindestens 70 % Identität mit der Aminosäuresequenz:
WMGPASMAMVAAAQP (SEQ ID NO: 3).

15. Immunoassay-Kit nach Anspruch 14, wobei das Kit mindestens ein einzelnes Peptid, mindestens zwei Peptide, mindestens drei Peptide, mindestens vier Peptide, mindestens fünf Peptide, mindestens sechs Peptide, mindestens sieben Peptide, mindestens acht Peptide, mindestens neun Peptide, mindestens zehn Peptide, mindestens elf Peptide, mindestens zwölf Peptide oder mindestens dreizehn Peptide, mindestens vierzehn Peptide, umfasst, wobei mindestens eines der Peptide 9 bis 18 Aminosäuren lang ist und eine Aminosäuresequenz umfasst, die mindestens 70% Identität mit der Aminosäuresequenz WMGPASMAMVAAAQP (SEQ ID NO: 3) aufweist oder aus der in SEQ ID NO:3 dargestellten Aminosäuresequenz besteht, und wobei das andere Peptid oder die anderen Peptide 9 bis 18 Aminosäuren lang ist/sind und jeweils unabhängig voneinander eine Aminosäuresequenz umfassen, die mindestens 70 % Identität mit der in SEQ ID NO: 1, SEQ ID NO: 2, SEQ ID Nr. 6, SEQ ID Nr. 7, SEQ ID Nr. 11, SEQ ID Nr. 14, SEQ ID Nr. 4, SEQ ID Nr. 15, SEQ ID Nr. 8, SEQ ID Nr. 13, SEQ ID Nr. 12, SEQ ID Nr. 5 oder SEQ ID Nr. 10 dargestellten Aminosäuresequenz aufweist oder aus der in SEQ ID NO: 1, SEQ ID NO: 2, SEQ ID Nr. 6, SEQ ID Nr. 7, SEQ ID Nr. 11, SEQ ID Nr. 14, SEQ ID Nr. 4, SEQ ID Nr. 15, SEQ ID Nr. 8, SEQ ID Nr. 13, SEQ ID Nr. 12, SEQ ID Nr. 5 oder SEQ ID Nr. 10 dargestellten Aminosäuresequenz bestehen, optional wobei das Peptid auf einem festen Träger immobilisiert ist.

## Revendications

1. Procédé de détection d'une réponse immunitaire à *Mycobacterium tuberculosis* chez un sujet, le procédé comprenant les étapes consistant à :
mettre en contact un échantillon biologique comprenant des cellules T provenant d'un sujet soupçonné d'avoir une réponse immunitaire à *Mycobacterium tuberculosis* avec au moins un peptide long de 8 à 18 acides aminés comprenant une séquence d'acides aminés présentant au moins 70 % d'identité avec la séquence d'acides aminés :
WMGPASMAMVAAAQP (SEQ ID NO: 3) ; et
détecter la présence ou l'absence de lymphocytes T dans l'échantillon biologique qui reconnaissent spécifiquement la séquence d'acides aminés du au moins un peptide.

2. Procédé selon la revendication 1, dans lequel le au moins un peptide est un peptide unique comprenant une séquence d'acides aminés présentant au moins 70 % d'identité avec la séquence d'acides aminés présentée dans SEQ ID NO : 3, ou dans lequel le au moins un peptide est un peptide unique constitué de la séquence d'acides aminés présentée dans SEQ ID NO : 3.

3. Procédé selon la revendication 1, dans lequel le procédé comprend la mise en contact de l'échantillon biologique avec au moins deux peptides, au moins trois peptides, au moins quatre peptides, au moins cinq peptides, au moins six peptides, au moins sept peptides, au moins huit peptides, au moins neuf peptides, au moins dix peptides, au moins onze peptides, au moins douze peptides, au moins treize peptides ou quatorze peptides, dans lequel au moins un desdits peptides présente une longueur de 8 à 18 acides aminés et comprend une séquence d'acides aminés présentant au moins 70% d'identité avec la séquence d'acides aminés : WMGPASMAMVAAAQP (SEQ ID NO: 3) ou consiste en la séquence d'acides aminés définie dans SEQ ID NO : 3, et dans lequel le ou les autres peptides présentent une longueur de 8 à 18 acides aminés et comprennent chacun indépendamment une séquence d'acides aminés présentant au moins 70 % d'identité avec la séquence d'acides aminés présentée dans SEQ ID NO :1, SEQ ID NO :2, SEQ ID NO :6, SEQ ID NO :7, SEQ ID NO :11, SEQ ID NO : 14, SEQ ID NO : 4, SEQ ID NO : 15, SEQ ID NO : 8, SEQ ID NO : 13, SEQ ID NO : 12, SEQ ID NO : 5 ou SEQ ID NO : 10 ou QTVEDEARRMW (SEQ ID NO :9), ou consistent en la séquence d'acides aminés présente dans SEQ ID NO :1, SEQ ID NO :2, SEQ ID NO :6, SEQ ID NO :7, SEQ ID NO :11, SEQ ID NO : 14, SEQ ID NO : 4, SEQ ID NO : 15, SEQ ID NO : 8, SEQ ID NO : 13, SEQ ID NO : 12, SEQ ID NO : 5 ou SEQ ID NO : 10.

4. Procédé selon l'une quelconque des revendications 1 à 3, dans lequel l'échantillon biologique provenant du sujet est du sang, des cellules mononucléées ce sang périphérique isolées ou des cellules mononucléées isolées.

5. Procédé selon l'une quelconque des revendications 1 à 4, dans lequel l'échantillon biologique comprend des cellules présentant un antigène.

6. Procédé selon l'une quelconque des revendications 1 à 5, dans lequel les lymphocytes T sont des lymphocytes T CD8+.

7. Procédé selon la revendication 6, dans lequel la détection comprend une mesure d'une sécrétion d'une cytokine à partir des lymphocytes T CD8+ et/ou une mesure d'une quantité d'une cytokine à l'intérieur des lymphocytes T CD8+.

8. Procédé selon la revendication 7, dans lequel la cytokine est l'interféron-y, le TNF et/ou l'IP-10.

9. Procédé selon l'une quelconque des revendications 6 à 8, dans lequel la détection comprend une mise en contact des lymphocytes T CD8+ avec des complexes de CMH de Classe I tétramérique/peptide qui s'associent spécifiquement à une protéine de surface de lymphocyte T CD8+.

10. Procédé selon l'une quelconque des revendications 1 à 9, dans lequel la détection comprend une réalisation d'un dosage d'immunospot lié à une enzyme ou d'un dosage d'immunosorbant lié à une enzyme, facultativement dans lequel le dosage détecte l'interférony ou l'IP-10.

11. Au moins un peptide long de 9 à 18 acides aminés pour utilisation dans un procédé de diagnostic d'une réponse immunitaire à *Mycobacterium tuberculosis* in vivo, le procédé comprenant les étapes consistant à :
administrer dans la peau d'un sujet soupçonné d'avoir une réponse immunitaire à *Mycobacterium tuberculosis* une quantité efficace du au moins un peptide long de 9 à 18 acides aminés, comprenant une séquence d'acides aminés présentant au moins 70 % d'identité avec la séquence d'acides aminés :
WMGPASMAMVAAAQP (SEQ ID NO: 3) ; et
détecter la présence de lymphocytes T qui reconnaissent spécifiquement la séquence d'acides aminés du peptide chez le sujet.

12. Peptide long d'au moins 9 à 18 acides aminés pour utilisation selon la revendication 11, dans lequel le procédé comprend l'administration dans la peau du sujet d'une quantité efficace d'un seul peptide, ou d'au moins deux peptides, d'au moins trois peptides, d'au moins quatre peptides, d'au moins cinq peptides, d'au moins six peptides, d'au moins sept peptides, d'au moins huit peptides, d'au moins neuf peptides, d'au moins dix peptides, d'au moins onze peptides, d'au moins douze peptides, d'au moins treize peptides ou de quatorze peptides, dans lequel au moins un desdits peptides présente une longueur de 9 à 18 acides aminés et comprend une séquence d'acides aminés présentant au moins 70 % d'identité avec la séquence d'acides aminés : WMGPASMAMVAAAQP (SEQ ID NO: 3) ou consiste en la séquence d'acides aminés définie dans SEQ ID NO : 3, et dans lequel le ou les autres peptides présentent une longueur de 9 à 18 acides aminés et comprennent chacun indépendamment une séquence d'acides aminés présentant au moins 70 % d'identité avec la séquence d'acides aminés présentée dans SEQ ID NO :1, SEQ ID NO :2, SEQ ID NO :6, SEQ ID NO :7, SEQ ID NO :11, SEQ ID NO : 14, SEQ ID NO : 4, SEQ ID NO : 15, SEQ ID NO : 8, SEQ ID NO : 13, SEQ ID NO : 12, SEQ ID NO : 5 ou SEQ ID NO : 10, ou consistent en la séquence d'acides aminés présentée dans SEQ ID NO :1, SEQ ID NO :2, SEQ ID NO :6, SEQ ID NO :7, SEQ ID NO :11, SEQ ID NO : 14, SEQ ID NO : 4, SEQ ID NO : 15, SEQ ID NO : 8, SEQ ID NO : 13, SEQ ID NO : 12, SEQ ID NO : 5 ou SEQ ID NO : 10, facultativement dans lequel les lymphocytes T sont des lymphocytes T CD8+.

13. Peptide long d'au moins 9 à 18 acides aminés pour utilisation selon la revendication 11 ou 12, dans lequel la détection de la présence ou de l'absence de lymphocytes T comprend une détection d'une réaction d'hypersensibilité de type retardé, facultativement dans lequel la réaction d'hypersensibilité de type retardé est mesurée en mesurant une rougeur, un gonflement ou une induration de la peau au niveau ou à proximité du site d'administration.

14. Kit de dosage immunologique pour détecter la présence de lymphocytes T dans un échantillon biologique d'un sujet ou au niveau d'un site d'administration intradermique chez le sujet indiquant une réponse immunitaire à *Mycobacterium tuberculosis* chez le sujet, le kit comprenant au moins un peptide long de 9 à 18 acides aminés comprenant une séquence d'acides aminés présentant au moins 70 % d'identité avec la séquence d'acides aminés :
WMGPASMAMVAAAQP (SEQ ID NO: 3).

15. Kit de dosage immunologique selon la revendication 14, dans lequel le kit comprend au moins un peptide unique, au moins deux peptides, au moins trois peptides, au moins quatre peptides, au moins cinq peptides, au moins six peptides, au moins sept peptides, au moins huit peptides, au moins neuf peptides, au moins dix peptides, au moins onze peptides, au moins douze peptides ou au moins treize peptides, au moins quatorze peptides, dans lequel au moins un desdits peptides présente une longueur de 9 à 18 acides aminés et comprend une séquence d'acides aminés présentant au moins 70% d'identité avec la séquence d'acides aminés : WMGPASMAMVAAAQP (SEQ ID NO: 3) ou consiste en la séquence d'acides aminés définie dans SEQ ID NO : 3, et dans lequel le ou les autres peptides présentent une longueur de 9 à 18 acides aminés et comprennent chacun indépendamment une séquence d'acides aminés présentant au moins 70 % d'identité avec la séquence d'acides aminés présentée dans SEQ ID NO :1, SEQ ID NO :2, SEQ ID NO :6, SEQ ID NO :7, SEQ ID NO :11, SEQ ID NO : 14, SEQ ID NO : 4, SEQ ID NO : 15, SEQ ID NO : 8, SEQ ID NO : 13, SEQ ID NO : 12, SEQ ID NO : 5 ou SEQ ID NO : 10 ou consistent en la séquence d'acides aminés présente dans SEQ ID NO :1, SEQ ID NO :2, SEQ ID NO :6, SEQ ID NO :7, SEQ ID NO :11, SEQ ID NO : 14, SEQ ID NO : 4, SEQ ID NO : 15, SEQ ID NO : 8, SEQ ID NO : 13, SEQ ID NO : 12, SEQ ID NO : 5 ou SEQ ID NO : 10, facultativement dans lequel le peptide est immobilisé sur un support solide.
